(19)

Europäisches
Patentamt
European
Patent Office
Office européen
des brevets

(11)     **EP 3 574 020 B1**

(12)        **EUROPEAN PATENT SPECIFICATION**

(45) Date of publication and mention
of the grant of the patent:
**15.05.2024   Bulletin 2024/20**

(21) Application number: **18835520.0**

(22) Date of filing: **18.07.2018**

(51) International Patent Classification (IPC):
*C07K 16/46* (2006.01)     *A61K 39/395* (2006.01)
*A61K 47/68* (2017.01)     *A61K 49/00* (2006.01)
*A61K 51/10* (2006.01)     *C07K 16/18* (2006.01)
*C12N 15/13* (2006.01)     *C12N 5/16* (2006.01)
*G01N 33/53* (2006.01)

(52) Cooperative Patent Classification (CPC):
**C07K 16/18; A61P 25/28; G01N 33/6896;**
A61K 2039/505; C07K 2317/24; C07K 2317/92;
G01N 2333/4709

(86) International application number:
**PCT/CA2018/050875**

(87) International publication number:
**WO 2019/014768 (24.01.2019 Gazette 2019/04)**

(54) **ANTIBODIES TO AMYLOID BETA**

ANTIKÖRPER GEGEN AMYLOID BETA

ANTICORPS ANTI-BÊTA-AMYLOÏDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.07.2017   PCT/CA2017/050866
09.11.2017   US 201715808842
25.01.2018   US 201862622126 P**

(43) Date of publication of application:
**04.12.2019   Bulletin 2019/49**

(73) Proprietors:
• **The University of British Columbia
Vancouver, British Columbia V6T 1Z3 (CA)**
• **ProMIS Neurosciences Inc.
Toronto, ON M4S 3E2 (CA)**

(72) Inventors:
• **CASHMAN, Neil R.
Vancouver, British Columbia V6S 1M8 (CA)**
• **PLOTKIN, Steven S.
Vancouver, British Columbia V5Z 1C3 (CA)**
• **KAPLAN, Johanne
Sherborn, Massachusetts 01770 (US)**
• **SILVERMAN, Judith Maxwell
Whistler, British Columbia V0N 1B4 (CA)**

• **GIBBS, Ebrima
Port Moody, British Columbia V3H 5J5 (CA)**

(74) Representative: **Hoggins, Mark Andrew
Symbiosis IP Limited
The Innovation Centre
217 Portobello
Sheffield S1 4DP (GB)**

(56) References cited:
**WO-A1-2017/025918     WO-A1-2017/079833
WO-A1-2018/014126     US-A1- 2018 125 920**

• **Anonymous: "Antitope - Antibody
huminization", , 9 March 2016 (2016-03-09),
XP055256546, Retrieved from the Internet:
URL:http://www.antitope.com/antibody-human
ization [retrieved on 2016-03-09]**
• **"AvantGen's Antibody Humanization and
Discovery Technologies", AVANTGEN, 27 July
2009 (2009-07-27), XP055127073,**
• **Anonymous: "Antibody Humanization | Antibody
Engineering", , 9 March 2016 (2016-03-09),
XP055256758, Retrieved from the Internet:
URL:https://lakepharma.com/productlist.php ?c
ategory=2&secondary=3 [retrieved on
2016-03-09]**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

- **PANKA D J ET AL: "VARIABLE REGION FRAMEWORK DIFFERENCES RESULT IN DECREASED OR INCREASED AFFINITY OF VARIANT ANTI-DIGOXIN ANTIBODIES", PROCEEDINGS OF THE NATIONAL ACADEMY OF SCIENCES, NATIONAL ACADEMY OF SCIENCES, US, vol. 85, no. 9, 1 May 1988 (1988-05-01), pages 3080-3084, XP000611718, ISSN: 0027-8424, DOI: 10.1073/PNAS.85.9.3080**
- **STEPHEN I. RUDNICK ET AL: "Affinity and Avidity in Antibody-Based Tumor Targeting", CANCER BIOTHERAPY & RADIOPHARMACEUTICALS, vol. 24, no. 2, 1 April 2009 (2009-04-01), pages 155-161, XP055069540, ISSN: 1084-9785, DOI: 10.1089/cbr.2009.0627**

Remarks:

The complete document including Reference Table(s) and the Sequence Listing(s) can be downloaded from the EPO website

**Description**

**Field**

The present disclosure relates to humanized antibodies that are selective for Amyloid beta (A-beta or Aβ) oligomers as well as compositions and uses thereof.

**Background**

Amyloid-beta (A-beta), which exists as a 36-43 amino acid peptide, is a product released from amyloid precursor protein (APP) by the enzymes β and γ secretase. In AD patients, A-beta can be present in soluble monomers, insoluble fibrils and soluble oligomers. In monomer form, A-beta exists as a predominantly unstructured polypeptide chain. In fibril form, A-beta can aggregate into distinct morphologies, often referred to as strains. Several of these structures have been determined by solid-state NMR.

For, example, structures for several strains of fibrils are available in the Protein Data Bank (PDB), a crystallographic database of atomic resolution three dimensional structural data, including a 3-fold symmetric Aβ structure (PDB entry, 2M4J); a two-fold symmetric structure of Aβ-40 monomers (PDB entry 2LMN), and a single-chain, parallel in-register structure of Aβ-42 monomers (PDB entry 2MXU).

The structure of 2M4J is reported in Lu et al [8], and the structure of 2MXU is reported in Xiao et al [9]. The structure of 2LMN is reported in Petkova et al [10].

A-beta oligomers have been shown to kill cell lines and neurons in culture and block a critical synaptic activity that subserves memory, referred to as long term potentiation (LTP), in slice cultures and living animals.

The structure of the oligomer has not been determined to date. Moreover, NMR and other evidence indicates that the oligomer exists not in a single well-defined structure, but in a conformationally-plastic, malleable structural ensemble with limited regularity. Moreover, the concentration of toxic oligomer species is far below either that of the monomer or fibril (estimates vary but are on the order of 1000-fold below or more), making this target elusive.

Antibodies that bind A-beta have been described.

WO2009048538A2 titled USE OF ANTI-AMYLOID ANTIBODY IN OCULAR DISEASES discloses chimeric antibodies that recognize one or more binding sites on A-beta and are useful for the treatment for ocular diseases.

US9221812B2 titled COMPOUNDS FOR THE TREATMENT OF DISEASES ASSOCIATED WITH AMYLOID OR AMYLOID-LIKE PROTEINS describes pharmaceutical compositions and discontinuous antibodies that bind A-beta including an epitope between amino acid residues 12 to 24 for the treatment of amyloid-related diseases.

WO2003070760A2 titled ANTI-AMYLOID BETA ANTIBODIES AND THEIR USE discloses antibodies that recognize an A-beta discontinuous epitope, wherein the first region comprises the amino acid sequence AEFRHDSGY or a fragment thereof and wherein the second region comprises the amino acid sequence VHHQKLVFFAEDVG or a fragment thereof.

US20110171243A1 titled COMPOUNDS TREATING AMYLOIDOSES discloses a peptide mimotope capable of inducing the *in vivo* formation of antibodies that bind HQKLVFand/or HQKLVFFAED, and its use.

WO2008088983A1 and WO2001062801A2 disclose a pegylated antibody fragment that binds A-beta amino acids 13-28 and its use in treating A-beta related diseases. Solanezumab and Crenezumab bind amino acids 16-26 on A-beta. Crenezumab interacts with the monomer, oligomer and fibril. Midregion antibodies, including solanezumab (picomolar affinity) and crenezumab (nanomolar affinity), appear to preferentially bind monomeric A-beta [13].

WO2009149487A2 titled COMPOUNDS FOR TREATING SYMPTOMS ASSOCIATED WITH PARKINSON'S DISEASE describes compounds comprising a peptide having binding capacity for an antibody specific for an A-beta epitope such as EVHHQKL, HQKLVF and HQKLVFFAED.

The HHQK (SEQ ID NO: 7) domain is described as involved in plaque induction of neurotoxicity in human microglia, as described in Giulian D *et al.* [11] and Winkler *et al.* [12]. Non-antibody therapeutic agents that bind HHQK (SEQ ID NO: 7) have been disclosed for the treatment of protein folding diseases (US20150105344A1, WO2006125324A1).

U.S. patents 5,766,846; 5,837,672; and 5,593,846 describe the production of murine monoclonal antibodies to the central domain of the Aβ peptide. WO 01/62801 describes antibodies that bind A-beta between amino acids 13-28. WO2004071408 discloses humanized antibodies.

WO2009086539A2 titled TREATMENT AND PROPHYLAXIS OF AMYLOIDOSIS is directed to Amyloidosis and amyloid light chain amyloidosis, by administering peptides comprising neoepitopes, such as amyloid protein A (AA) fragments from a C-terminal region of AA, and antibodies specific for neoepitopes of aggregated amyloid proteins, for example, antibodies specific for the C-terminal region of AA fibrils.

WO2003070760 titled ANTI-AMYLOID BETA ANTIBODIES AND THEIR USE is directed towards antibody molecules capable of specifically recognizing two regions of the R-A4 peptide, wherein the first region comprises the amino acid sequence AEFRHDSGY or a fragment thereof and wherein the second region comprises the amino acid sequence VHHAEDVFFAEDVG or a fragment thereof.

WO2006066089 titled HUMANIZED AMYLOID BETA ANTIBODIES FOR USE IN IMPROVING COGNITION is directed to improved agents and methods for treatment of diseases associated with beta amyloid and in particular to the identification and characterization of a monoclonal antibody, 12A11, that specifically binds to Aβ peptide and is effective at reducing plaque burden associated with amyloidogenic disorders (e.g., AD).

WO2007068429 titled ANTIBODIES AGAINST AMYLOID BETA 4 WITH GLYCOSYLATED IN THE VARIABLE REGION is directed to a purified antibody molecule preparation being characterized in that at least one antigen binding site comprises a glycosylated asparagine (Asn) in the variable region of the heavy chain ($V_H$).

WO 03/016466 is directed variant 266 antibodies that are engineered to lack an N-glycosylation site within the CDR2 of the heavy chain, pharmaceutical compositions thereof, and polynucleotide sequences, vectors, and transformed cells useful to express the variant antibodies. The variants are described to sequester soluble A-beta peptide from human biological fluids and specifically bind an epitope contained within position 13-28 of the amyloid beta peptide.

Yu et al. describes a hexavalent foldable Aβ1-15 (6Aβ15) fused to PADRE or toxin-derived carrier proteins. Wang et al 2016 report that peripheral administration of this antibody mitigates Alzheimer's disease like pathology and cognitive decline in a transgenic animal of aged Alzheimer Disease [14], [15].

WO 2017/079833 A1 describes murine monoclonal antibodies raised against a cyclic HHQK peptide.

Antibodies that preferentially or selectively bind A-beta oligomers over monomers or over fibrils or over both monomers and fibrils are desirable.

## Summary

The present invention, in its various aspects, is as set out in the accompanying claims.

An aspect includes an humanized antibody comprising a heavy chain variable region and a light chain variable region, wherein: the heavy chain variable region comprises an amino acid sequence as set forth in SEQ ID NO: 46; and the light chain variable region comprises an amino acid sequence as set forth in SEQ ID NO: 66; and the humanized antibody binds amyloid beta oligomers.

In an embodiment, the antibody is an antibody binding fragment of an antibody described herein selected from Fab, Fab', F(ab')2, scFv, dsFv, ds-scFv, dimers, minibodies, diabodies, and multimers thereof.

An aspect includes an immunoconjugate comprising the humanized antibody described herein and a detectable label or cytotoxic agent.

An aspect includes a composition comprising the antibody described herein, or the immunoconjugate described herein and a diluent.

An aspect includes a nucleic acid molecule encoding a proteinaceous portion the antibody described herein.

An aspect includes a vector comprising the nucleic acid described herein.

An aspect includes a cell expressing an antibody described herein.

An aspect includes the immunoconjugate described herein for use in measuring a level of A-beta in a subject at risk or suspected of having or having AD, wherein the antibody is conjugated to a detectable label.

In an embodiment, the label is a positron emitting radionuclide.

An aspect includes the antibody or immunoconjugate described herein, or a pharmaceutical composition comprising the antibody or immunoconjugate according to the invention for use in treating AD, Lewy body dementia and/or cerebral amyloid angiopathy in a subject in need thereof, preferably wherein the antibody or immunoconjugate is for administration directly to the brain or other portion of the CNS.

In an embodiment, a biological sample from the subject to be treated is assessed for the presence or levels of A-beta using an antibody described herein.

Other features and advantages of the present disclosure will become apparent from the following detailed description.

Any references in the description to methods of treatment refer to the compounds, pharmaceutical compositions and medicaments of the present invention for use in a method for treatment of the human (or animal) body by therapy (or for diagnosis).

## Brief description of the drawings

The present disclosure will now be described in relation to the drawings in which:

FIG. 1 is a graph reporting effect of antibodies on propagation of A-beta oligomers in vitro.
FIG. 2 is a graph showing that the humanized antibody shows significantly better binding to the toxic oligomer enriched LMW fraction of AD extract.

FIGs. 3A to 3F are a series of brain sections stained with various Abeta and control antibodies.

FIG 4A is a trace an AD soluble brain extract separated

FIGs. 4B to 4D are graphs showing the levels of A-beta detected in LMW and HMW fractions.

FIG. 5A is a graph showing levels of human IgG detectable in brain and plasma at 24hrs after injection of anti-Abeta antibodies.

FIG. 5B is a graph showing levels of anti-Abeta antibody CNS penetrance.

FIGs. 5C and 5D show levels of IgG detected in brain and plasma or non-transgenic and App/PS1 mice 1-21 days after injection of anti-Abeta antibodies.

## Detailed description of the Disclosure

Provided herein are humanized antibodies comprising a heavy chain variable region and a light chain variable region, wherein: the heavy chain variable region comprises an amino acid sequence as set forth in SEQ ID NO: 46; the light chain variable region comprises an amino acid sequence as set forth in SEQ ID NO: 66; and the humanized antibody binds amyloid beta oligomers, immunotherapeutic compositions thereof and methods of use thereof. Said antibodies may target epitopes preferentially accessible in toxic oligomeric species of A-beta, including oligomeric species associated with Alzheimer's disease (AD).

As shown in the Examples, antibodies raised using a cyclic peptide comprising HHQK (SEQ ID NO: 7), preferentially bound oligomeric A-beta and/or selectively bound the cyclic peptide compared to a linear peptide of the same sequence (e.g. corresponding linear sequence). Experimental results are described and identify epitope-specific and conformationally selective antibodies that bind synthetic oligomer selectively compared to synthetic monomers, bind CSF from AD patients preferentially over control CSF and/or bind soluble brain extract from AD patients preferentially over control soluble brain extract. Further staining of AD brain tissue identified antibodies that show no or negligible plaque binding and in vitro studies found that the antibodies inhibited Aβ oligomer propagation and aggregation.

As further shown in the Examples, humanized antibody 301-17 having a heavy chain variable region comprising an amino acid sequence as set forth in SEQ ID NO: 46 and a light chain variable region comprising an amino acid sequence as set forth in SEQ ID NO: 66 showed improved characteristics such as improved binding of oligomeric A-beta compared to the mouse monoclonal antibody. The humanized antibodies also had improved specificity compared to the parental monoclonal.

For example, the humanized antibody has increased binding, such as 50% increased or up to 200% increased (or any number in between) compared to mouse monoclonal antibody, to oligomeric A-beta, optionally oligomeric Abeta present in low molecular weight fractions of samples such as brain extracts or other biological samples.

## I. Definitions

As used herein, the term 'A-beta' may alternately be referred to as 'amyloid beta', 'amyloid β', A-beta, A-beta or 'Aβ'. Amyloid beta is a peptide of 36-43 amino acids and includes all wildtype and mutant forms of all species, particularly human A-beta. A-beta40 refers to the 40 amino acid form; A-beta42 refers to the 42 amino acid form, etc. The amino acid sequence of human wildtype A-beta42 is shown in SEQ ID NO: 73.

As used herein, the term "A-beta monomer" herein refers to any of the individual subunit forms of the A-beta (e.g. 1-40, 1-42, 1-43) peptide.

As used herein, the term "A-beta oligomer" herein refers to a plurality of any of the A-beta subunits wherein several (e.g. at least two) A-beta monomers are non-covalently aggregated in a conformationally-flexible, partially-ordered, three-dimensional globule of less than about 100, or more typically less than about 50 monomers. For example, an oligomer may contain 3 or 4 or 5 or more monomers. The term "A-beta oligomer" as used herein includes both synthetic A-beta oligomer and/or native A-beta oligomer. "Native A-beta oligomer" refers to A-beta oligomer formed in vivo, for example in the brain and CSF of a subject with AD.

As used herein, the term "A-beta fibril" refers to a molecular structure that comprises assemblies of non-covalently associated, individual A-beta peptides which show fibrillary structure under an electron microscope. The fibrillary structure is typically a "cross beta" structure; there is no theoretical upper limit on the size of multimers, and fibrils may comprise thousands or many thousands of monomers. Fibrils can aggregate by the thousands to form senile plaques, one of the primary pathological morphologies diagnostic of AD.

The term "HHQK" means the amino acid sequence histidine, histidine, glutamine, lysine, as shown in SEQ ID NO: 7. Depending on the context, the reference of the amino acid sequence can refer to a sequence in A-beta or an isolated peptide, such as the amino acid sequence of a cyclic compound.

The term "amino acid" includes all of the naturally occurring amino acids as well as modified L-amino acids. The atoms of the amino acid can include different isotopes. For example, the amino acids can comprise deuterium substituted for hydrogen nitrogen-15 substituted for nitrogen-14, and carbon-13 substituted for carbon-12 and other similar changes.

The term "antibody" as used herein is intended to include monoclonal antibodies, polyclonal antibodies, single chain, veneered, humanized and other chimeric antibodies and binding fragments thereof, including for example a single chain Fab fragment, Fab'2 fragment or single chain Fv fragment. The antibody may be from recombinant sources and/or produced in animals such as rabbits, llamas, sharks etc. Also included are human antibodies that can be produced in transgenic animals or using biochemical techniques or can be isolated from a library such as a phage library. Humanized or other chimeric antibodies may include sequences from one or more than one isotype or class or species.

The phrase "isolated antibody" refers to antibody produced in vivo or in vitro that has been removed from the source that produced the antibody, for example, an animal, hybridoma or other cell line (such as recombinant insect, yeast or bacteria cells that produce antibody). The isolated antibody is optionally "purified", which means at least: 80%, 85%, 90%, 95%, 98% or 99% purity.

The term "binding fragment" as used herein to a part or portion of an antibody or antibody chain comprising fewer amino acid residues than an intact or complete antibody or antibody chain and which binds the antigen or competes with intact antibody. Exemplary binding fragments include without limitations Fab, Fab', F(ab)2, scFv, dsFv, ds-scFv, dimers, nanobodies, minibodies, diabodies, and multimers thereof. Fragments can be obtained via chemical or enzymatic treatment of an intact or complete antibody or antibody chain. Fragments can also be obtained by recombinant means. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be constructed by recombinant expression techniques.

The terms "IMGT numbering" or "ImMunoGeneTics database numbering", which are recognized in the art, refer to a system of numbering amino acid residues which are more variable (i.e. hypervariable) than other amino acid residues in the heavy and light chain variable regions of an antibody, or antigen binding portion thereof.

As used herein, the term "conformational epitope" refers to an epitope where the epitope amino acid sequence has a particular three-dimensional structure wherein at least an aspect of the three-dimensional structure not present or less likely to be present in another form for example a corresponding linear peptide or A-beta monomer and is specifically and/or selectively recognized by the cognate antibody. Antibodies which specifically bind a conformation-specific epitope recognize the spatial arrangement of one or more of the amino acids of that conformation-specific epitope. For example, an HHQK (SEQ ID NO: 7) conformational epitope refers to an epitope of HHQK (SEQ ID NO:7) that is recognized by antibodies selectively, for example at least 2 fold, 3 fold, 5 fold, 10 fold, 50 fold, 100 fold, 250 fold, 500 fold or 1000 fold or greater more selectivity as compared to antibodies raised using linear HHQK (SEQ ID NO: 7). When an antibody is said to selectively bind an epitope such as a conformational epitope, such as HHQK (SEQ ID NO: 7), what is meant is that the antibody preferentially binds one or more particular conformations containing the specified residues or a part thereof with greater affinity than it binds said residues in another conformation. For example, when an antibody is said to selectively bind a cyclopeptide comprising HHQK or related epitope relative to a corresponding linear peptide, the antibody binds the cyclopeptide with at least a 2 fold greater affinity than it binds the linear peptide. Similarly, when an antibody is said to selectively bind oligomeric A-beta, the antibody binds the oligomeric species with at least a 2 fold greater affinity than it binds A-beta monomer and/or plaque fibrils.

The term "no or negligible plaque binding" or "lacks or has negligible plaque binding" as used herein with respect to an antibody means that the antibody does not show typical plaque morphology staining on immunohistochemistry (e.g. in situ, optionally as compared to plaque staining seen with A-beta antibody 6E10) and the level of staining is comparable to or no more than 2 fold the level seen with an IgG negative (e.g. irrelevant) isotype control.

The term "Isolated peptide" refers to peptide that has been produced, for example, by recombinant or synthetic techniques, and removed from the source that produced the peptide, such as recombinant cells or residual peptide synthesis reactants. The isolated peptide is optionally "purified", which means at least: 80%, 85%, 90%, 95%, 98% or 99% purity and optionally pharmaceutical grade purity.

The term "detectable label" as used herein refers to moieties such as peptide sequences (such a myc tag, HA-tag, V5-tag or NE-tag), fluorescent proteins that can be appended or introduced into a peptide or compound described herein and which is capable of producing, either directly or indirectly, a detectable signal. For example, the label may be radio-opaque, positron-emitting radionuclide (for example for use in PET imaging), or a radioisotope, such as $^3$H, $^{13}$N, $^{14}$C, $^{18}$F, $^{32}$P, $^{35}$S, $^{123}$I, $^{125}$I, $^{131}$I; a fluorescent (fluorophore) or chemiluminescent (chromophore) compound, such as fluorescein isothiocyanate, rhodamine or luciferin; an enzyme, such as alkaline phosphatase, beta-galactosidase or horseradish peroxidase; an imaging agent; or a metal ion. The detectable label may be also detectable indirectly for example using secondary antibody.

The term "epitope" as commonly used means an antibody binding site, typically a polypeptide segment, in an antigen that is specifically recognized by the antibody. As used herein "epitope" can also refer to the amino acid sequences or part thereof identified on A-beta using the collective coordinates method described. For example an antibody generated against an isolated peptide corresponding to a cyclic compound comprising the identified target region HHQK SEQ ID NO:7), recognizes part or all of said epitope sequence. An epitope is "accessible" in the context of the present specification when it is accessible to binding by an antibody.

The term "greater affinity" as used herein refers to a relative degree of antibody binding where an antibody X binds to target Y more strongly ($K_{on}$) and/or with a smaller dissociation constant ($K_{off}$) than to target Z, and in this context antibody X has a greater affinity for target Y than for Z. Likewise, the term "lesser affinity" herein refers to a degree of antibody binding where an antibody X binds to target Y less strongly and/or with a larger dissociation constant than to target Z, and in this context antibody X has a lesser affinity for target Y than for Z. The affinity of binding between an antibody and its target antigen, can be expressed as $K_A$ equal to $1/K_D$ where $K_D$ is equal to $k_{on}/k_{off}$. The $k_{on}$ and $k_{off}$ values can be measured using surface plasmon resonance technology, for example using a Molecular Affinity Screening System (MASS-1) (Sierra Sensors GmbH, Hamburg, Germany). An antibody that is selective for a conformation presented in a cyclic compound optional a cyclic peptide for example has a greater affinity for the cyclic compound (e.g. cyclic peptide) compared to a corresponding sequence in linear form (e.g. the sequence non-cyclized).

The term "corresponding linear compound" with regard to a cyclic compound refers to a compound, optionally a peptide, comprising or consisting of the same sequence or chemical moieties as the cyclic compound but in linear (i.e. non-cyclized) form, for example having properties as would be present in solution of a linear peptide. For example, the corresponding linear compound can be the synthesized peptide that is not cyclized.

As used herein "specifically binds" in reference to an antibody means that the antibody recognizes an epitope sequence and binds to its target antigen with a minimum affinity. For example a multivalent antibody binds its target with a $K_D$ of at least 1e-6, at least 1e-7, at least 1e-8, at least 1e-9, or at least 1e-10. Affinities greater than at least 1e-8 may be preferred. For example the $K_D$ may be in the nanomolar range or the picomolar range. An antigen binding fragment such as Fab fragment comprising one variable domain, may bind its target with a 10 fold or 100 fold less affinity than a multivalent interaction with a non-fragmented antibody.

The term "selectively binds" as used herein with respect to an antibody that selectively binds a form of A-beta (e.g. fibril, monomer or oligomer) or a cyclic compound means that the antibody binds the form with at least 2 fold, at least 3 fold, or at least 5 fold, at least 10 fold, at least 100 fold, at least 250 fold, at least 500 fold or at least 1000 fold or more greater affinity. Accordingly an antibody that is more selective for a particular conformation (e.g. oligomer) preferentially binds the particular form of A-beta with at least 2 fold etc. greater affinity compared to another form and/or a linear peptide.

The term "animal" or "subject" as used herein includes all members of the animal kingdom including mammals, optionally including or excluding humans.

A "conservative amino acid substitution" as used herein, is one in which one amino acid residue is replaced with another amino acid residue without abolishing the protein's desired properties. Suitable conservative amino acid substitutions can be made by substituting amino acids with similar hydrophobicity, polarity, and R-chain length for one another. Examples of conservative amino acid substitution include:

| Conservative Substitutions | |
| --- | --- |
| Type of Amino Acid | Substitutable Amino Acids |
| Hydrophilic | Ala, Pro, Gly, Glu, Asp, Gln, Asn, Ser, Thr |
| Sulphydryl | Cys |
| Aliphatic | Val, Ile, Leu, Met |
| Basic | Lys, Arg, His |
| Aromatic | Phe, Tyr, Trp |

The term "sequence identity" as used herein refers to the percentage of sequence identity between two polypeptide sequences or two nucleic acid sequences. To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino acid or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=number of identical overlapping positions/total number of positions.times.100%). In one embodiment, the two sequences are the same length. The determination of percent identity between two sequences can also be accomplished using a mathematical algorithm. A preferred, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST and XBLAST programs of Altschul et al., 1990, J. Mol. Biol. 215:403. BLAST nucleotide searches can be performed with the NBLAST nucleotide program parameters set, e.g., for score=100, word length=12 to obtain nucleotide sequences homologous to a nucleic acid molecules of the present application. BLAST protein searches can be performed with the XBLAST program parameters set, e.g., to score-50, word length=3 to obtain amino acid sequences homologous to a protein molecule described herein. To obtain gapped alignments for comparison purposes, Gapped BLAST can be utilized as described in Altschul et al., 1997, Nucleic Acids Res. 25:3389-3402. Alternatively, PSI-BLAST can be used to perform an iterated search which detects distant relationships between molecules (Id.). When utilizing BLAST, Gapped BLAST, and PSI-Blast programs, the default parameters of the respective programs (e.g., of XBLAST and NBLAST) can be used (see, e.g., the NCBI website). Another preferred non-limiting example of a mathematical algorithm utilized for the comparison of sequences is the algorithm of Myers and Miller, 1988, CABIOS 4:11-17. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. The percent identity between two sequences can be determined using techniques similar to those described above, with or without allowing gaps. In calculating percent identity, typically only exact matches are counted.

For antibodies, percentage sequence identities can be determined when antibody sequences maximally aligned by IMGT or other (e.g. Kabat numbering convention). After alignment, if a subject antibody region (e.g., the entire mature variable region of a heavy or light chain) is being compared with the same region of a reference antibody, the percentage sequence identity between the subject and reference antibody regions is the number of positions occupied by the same amino acid in both the subject and reference antibody region divided by the total number of aligned positions of the two regions, with gaps not counted, multiplied by 100 to convert to percentage.

The term "nucleic acid sequence" as used herein refers to a sequence of nucleoside or nucleotide monomers consisting of naturally occurring bases, sugars and intersugar (backbone) linkages. The term also includes modified or substituted sequences comprising non-naturally occurring monomers or portions thereof. The nucleic acid sequences of the present application may be deoxyribonucleic acid sequences (DNA) or ribonucleic acid sequences (RNA) and may include naturally occurring bases including adenine, guanine, cytosine, thymidine and uracil. The sequences may also contain modified bases. Examples of such modified bases include aza and deaza adenine, guanine, cytosine, thymidine and uracil; and xanthine and hypoxanthine. The nucleic acid can be either double stranded or single stranded, and represents the sense or antisense strand. Further, the term "nucleic acid" includes the complementary nucleic acid sequences as well as codon optimized or synonymous codon equivalents. The term "isolated nucleic acid sequences" as used herein refers to a nucleic acid substantially free of cellular material or culture medium when produced by recombinant DNA techniques, or chemical precursors, or other chemicals when chemically synthesized. An isolated nucleic acid is also substantially free of sequences which naturally flank the nucleic acid (i.e. sequences located at the 5' and 3' ends of the nucleic acid) from which the nucleic acid is derived.

"Operatively linked" is intended to mean that the nucleic acid is linked to regulatory sequences in a manner which allows expression of the nucleic acid. Suitable regulatory sequences may be derived from a variety of sources, including bacterial, fungal, viral, mammalian, or insect genes. Selection of appropriate regulatory sequences is dependent on the host cell chosen and may be readily accomplished by one of ordinary skill in the art. Examples of such regulatory sequences include: a transcriptional promoter and enhancer or RNA polymerase binding sequence, a ribosomal binding sequence, including a translation initiation signal. Additionally, depending on the host cell chosen and the vector employed, other sequences, such as an origin of replication, additional DNA restriction sites, enhancers, and sequences conferring inducibility of transcription may be incorporated into the expression vector.

The term "vector" as used herein comprises any intermediary vehicle for a nucleic acid molecule which enables said nucleic acid molecule, for example, to be introduced into prokaryotic and/or eukaryotic cells and/or integrated into a genome, and include plasmids, phagemids, bacteriophages or viral vectors such as retroviral based vectors, Adeno Associated viral vectors and the like. The term "plasmid" as used herein generally refers to a construct of extrachromosomal genetic material, usually a circular DNA duplex, which can replicate independently of chromosomal DNA.

By "at least moderately stringent hybridization conditions" it is meant that conditions are selected which promote selective hybridization between two complementary nucleic acid molecules in solution. Hybridization may occur to all or a portion of a nucleic acid sequence molecule. The hybridizing portion is typically at least 15 (e.g. 20, 25, 30, 40 or 50) nucleotides in length. Those skilled in the art will recognize that the stability of a nucleic acid duplex, or hybrids, is determined by the Tm, which in sodium containing buffers is a function of the sodium ion concentration and temperature (Tm = 81.5°C -16.6 (Log10 [Na+]) + 0.41(%(G+C) -600/l), or similar equation). Accordingly, the parameters in the wash conditions that determine hybrid stability are sodium ion concentration and temperature. In order to identify molecules that are similar, but not identical, to a known nucleic acid molecule a 1% mismatch may be assumed to result in about a 1°C decrease in Tm, for example if nucleic acid molecules are sought that have a >95% identity, the final wash temperature will be reduced by about 5°C. Based on these considerations those skilled in the art will be able to readily select appropriate hybridization conditions. In preferred embodiments, stringent hybridization conditions are selected. By way of example the following conditions may be employed to achieve stringent hybridization: hybridization at 5x sodium chloride/sodium citrate (SSC)/5x Denhardt's solution/1.0% SDS at Tm - 5°C based on the above equation, followed by a wash of 0.2x SSC/0.1% SDS at 60°C. Moderately stringent hybridization conditions include a washing step in 3x SSC at 42°C. It is understood, however, that equivalent stringencies may be achieved using alternative buffers, salts and temperatures. Additional guidance regarding hybridization conditions may be found in: Current Protocols in Molecular Biology, John Wiley & Sons, N.Y., 2002, and in: Sambrook et al., Molecular Cloning: a Laboratory Manual, Cold Spring Harbor Laboratory Press, 2001.

The term "treating" or "treatment" as used herein and as is well understood in the art, means an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (whether partial or total), whether detectable or undetectable. "Treating" and "Treatment" can also mean prolonging survival as compared to expected survival if not receiving treatment. "Treating" and "treatment" as used herein also include prophylactic treatment. For example, a subject with early stage AD can be treated to prevent progression can be treated with a compound, antibody, immunogen, nucleic acid or composition described herein to prevent progression.

The term "administered" as used herein means administration of a therapeutically effective dose of a compound or composition of the disclosure to a cell or subject.

As used herein, the phrase "effective amount" means an amount effective, at dosages and for periods of time necessary to achieve a desired result. Effective amounts when administered to a subject may vary according to factors such as the disease state, age, sex, weight of the subject. Dosage regime may be adjusted to provide the optimum therapeutic response.

The term "pharmaceutically acceptable" means that the carrier, diluent, or excipient is compatible with the other components of the formulation and not substantially deleterious to the recipient thereof.

Compositions or methods "comprising" or "including" one or more recited elements may include other elements not specifically recited. For example, a composition that "comprises" or "includes" an antibody may contain the antibody alone or in combination with other ingredients.

In understanding the scope of the present disclosure, the term "consisting" and its derivatives, as used herein, are intended to be close ended terms that specify the presence of stated features, elements, components, groups, integers, and/or steps, and also exclude the presence of other unstated features, elements, components, groups, integers and/or steps.

The recitation of numerical ranges by endpoints herein includes all numbers and fractions subsumed within that range (e.g. 1 to 5 includes 1, 1.5, 2, 2.75, 3, 3.90, 4, and 5). It is also to be understood that all numbers and fractions thereof are presumed to be modified by the term "about." Further, it is to be understood that "a," "an," and "the" include plural referents unless the content clearly dictates otherwise. The term "about" means plus or minus 0.1 to 50%, 5-50%, or 10-40%, preferably 10-20%, more preferably 10% or 15%, of the number to which reference is being made.

Further, the definitions and embodiments described in particular sections are intended to be applicable to other embodiments herein described for which they are suitable as would be understood by a person skilled in the art. For example, in the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The singular forms of the articles "a," "an," and "the" include plural references unless the context clearly dictates otherwise. For example, the term "a compound" or "at least one compound" can include a plurality of compounds, including mixtures thereof.

## II. Antibodies and Nucleic acids

Disclosed herein are particular antibodies and uses thereof.

As demonstrated in the Examples, antibodies raised using cyclo(CGHHQKG) (SEQ ID NO: 12) were sequenced, selectively bound the cyclic compound relative to the corresponding linear peptide, selectively bound A-beta oligomer over monomer, and/or lacked appreciable plaque staining in AD tissue. Further said antibody was able to inhibit in vitro propagation of A-beta aggregation.

Accordingly an aspect includes an antibody comprising a light chain variable region and a heavy chain variable region, wherein the heavy chain variable region comprises an amino acid sequence as set forth in SEQ ID NO: 46; the light chain variable region comprises an amino acid sequence as set forth in SEQ ID NO: 66; and the humanized antibody binds amyloid beta oligomers.

For example, the antibody lacks binding a linear peptide comprising the sequence HHQK (SEQ ID NO: 7), optionally wherein the sequence of the linear peptide is a linear version of a cyclic sequence used to raise the antibody, optionally under conditions described in the Examples.

For example, the antibody specifically binds an epitope on A-beta as present in vivo, the epitope comprising or consisting HHQK (SEQ ID NO: 7), or a part thereof.

For example, the antibody does not specifically bind and/or is not selective for linear peptides consisting of HHQK (SEQ ID NO: 7). Selective binding can be measured using an ELISA or surface plasmon resonance measurement, as described herein.

For example, the antibody selectively binds a cyclic compound comprising HHQK (SEQ ID NO: 7) or a part thereof, optionally in the context of cyclo(CGHHQKG) (SEQ ID NO: 12) relative to a linear peptide comprising HHQK (SEQ ID NO: 7), optionally in the context of linear CGHHQKG (SEQ ID NO: 12). For example, in an embodiment the antibody selectively binds HHQK (SEQ ID NO: 7) in a cyclic conformation and has at least 2 fold, at least 5 fold, at least 10 fold at least 20 fold, at least 30 fold, at least 40 fold, at least 50 fold, at least 100 fold, at least 500 fold, at least 1000 fold more selective for HHQK (SEQ ID NO: 7) in the cyclic conformation compared to HHQK (SEQ ID NO: 7) in a linear compound such as a corresponding linear compound, for example as measured by ELISA or surface plasmon resonance, optionally using a method described herein.

For example, the antibody selectively binds a cyclic compound comprising the epitope sequence relative to linear peptide or a species of A-beta such as A-beta oligomer relative to monomer. In an embodiment, the selectivity is at least 2 fold, at least 3 fold, at least 5 fold, at least 10 fold, at least 20 fold, at least 30 fold, at least 40 fold, at least 50 fold, at least 100 fold, at least 500 fold, at least 1000 fold more selective for the cyclic compound and/or A-beta oligomer over a species of A-beta selected from A-beta monomer and/or A-beta fibril and/or linear HHQK (SEQ ID NO: 7), optionally linear CGHHQKG (SEQ ID NO: 12).

For example, the antibody is a monoclonal antibody. The production of monoclonals is described in the Examples.

To produce monoclonal antibodies, antibody producing cells (lymphocytes) can be harvested from a subject immunized with an immunogen described herein, and fused with myeloma cells by standard somatic cell fusion procedures thus immortalizing these cells and yielding hybridoma cells. Such techniques are well known in the art, (e.g. the hybridoma technique originally developed by Kohler and Milstein (Nature 256:495-497 (1975)) as well as other techniques such as the human B-cell hybridoma technique (Kozbor et al., Immunol.Today 4:72 (1983)), the EBV-hybridoma technique to produce human monoclonal antibodies (Cole et al., Methods Enzymol, 121 : 140-67 (1986)), and screening of combinatorial antibody libraries (Huse et al., Science 246:1275 (1989)). Hybridoma cells can be screened immunochemically for production of antibodies specifically reactive with the desired epitopes and the monoclonal antibodies can be isolated.

Specific antibodies, or antibody fragments, reactive against particular antigens or molecules, may also be generated by screening expression libraries encoding immunoglobulin genes, or portions thereof, expressed in bacteria with cell surface components. For example, complete Fab fragments, VH regions and FV regions can be expressed in bacteria using phage expression libraries (see for example Ward et al., Nature 41:544-546 (1989); Huse et al., Science 246:1275-1281 (1989); and McCafferty et al., Nature 348:552-554 (1990).

As demonstrated in the Examples, specific humanized antibodies are described. The humanization of antibodies from non-human species has been described in the literature. See for example EP-B1 0 239400 and Carter & Merchant 1997 (Curr Opin Biotechnol 8, 449-454, 1997). Humanized antibodies are also readily obtained commercially (eg. Scotgen Limited, 2 Holly Road, Twickenham, Middlesex, Great Britain.).

Humanized forms of rodent antibodies can be generated by CDR grafting (Riechmann et al. Nature, 332:323-327, 1988). In this approach the six CDR loops comprising the antigen binding site of the rodent monoclonal antibody are linked to corresponding human framework regions. CDR grafting often yields antibodies with reduced affinity as the amino acids of the framework regions may influence antigen recognition (Foote & Winter. J Mol Biol, 224: 487-499, 1992). To maintain the affinity of the antibody, it is often necessary to replace certain framework residues by site directed mutagenesis or other recombinant techniques and may be aided by computer modeling of the antigen binding site (Co et al. J Immunol, 152: 2968-2976, 1994).

The method described in the Examples can also be used.

The method can for example the variable region can be grafted into a human framework and for example the introduction of specific mutations can be introduced into the variable region outside of the CDRs.

Humanized forms of antibodies are optionally obtained by resurfacing (Pedersen et al. J Mol Biol, 235: 959-973, 1994). In this approach only the surface residues of a rodent antibody are humanized.

The disclosure also relates to an antibody that competes for binding to a cyclic peptide having sequence of SEQ ID NO: 12, and/or human A-beta optionally human A-beta oligomers with an antibody described herein.

The humanized antibody of the invention comprises a heavy chain variable region comprising: an amino acid sequence as set forth in SEQ ID NO: 46; and a light chain variable region comprising an amino acid sequence as set forth in SEQ ID NO: 66.

In an embodiment, the heavy chain variable region amino acid sequence is encoded by a nucleotide sequence as set forth in SEQ ID NO: 45; or a codon degenerate or optimized version thereof; and the light chain variable region amino acid sequence encoded is by a nucleotide sequence as set out in SEQ ID NO: 65 or a codon degenerate or optimized version thereof.

The heavy chain variable region comprises or consists of an amino acid sequence as set forth in SEQ ID NO: 46, and the light chain variable region comprises or consists of an amino acid sequence as set forth in SEQ ID NO: 66.

For example, the antibody is an antibody that competes for binding to a cyclic peptide having sequence to SEQ ID NO: 12, and/or human A-beta oligomers with an antibody comprising the heavy chain variable chain sequence of SEQ ID NO: 46, and the light chain variable region sequence of SEQ ID NO: 66.

For example, an antibody described herein comprises a constant region having i) an amino acid sequence as set forth in SEQ ID NO:70 and/or 72; ii) an amino acid sequence with at least 50%, at least 60%, at least 70%, at least 80%, or at least 90% sequence identity to any one of SEQ ID NO:70 and/or 72; or iii) a conservatively substituted amino acid sequence i).

For example, the heavy chain constant region amino acid sequence is encoded by a nucleotide sequence as set forth in SEQ ID NO: 69; or a codon degenerate or optimized version thereof; and/or the antibody comprises a light chain constant region amino acid sequence encoded by a nucleotide sequence as set out in SEQ ID NO:71, or a codon degenerate or optimized version thereof.

For example, the humanized antibody comprises the sequences of VH2 Vk5.

In a preferred embodiment of the invention said antibody is an IgG1, IgG2, IgG3 or IgG4 antibody and/or an antibody binding fragment, wherein said antibody binding fragment is selected from Fab, Fab', F(ab')2, scFv, dsFv, ds-scFv, dimers, nanobodies, minibodies, diabodies, and multimers thereof, preferably wherein the antibody binding fragment is a Fab fragment.

For example, the humanized antibody is a Fab fragment.

For example, the Fab fragment comprises VH2 Vk5.

In a further embodiment of the invention the humanized antibody according to the invention, comprises the CH1 and/or CL sequence or a part thereof of IgG4, preferably wherein the CH1 and/or CL sequence comprises SEQ ID NO: 70 or 72 or a part thereof, or a conservative variant thereof or a sequence with at least 50%, 60%, 70%, 89%, 90% or 95% sequence identity to SEQ ID NO: 70 and/or 72, preferably wherein the antibody comprises SEQ ID NO: 70 and/or 72, and/or CH1 and CH2 of SEQ ID NO: 70 or a conservative variant of any of the foregoing or a sequence with at least 50%, 60%, 70%, 89%, 90% or 95% sequence identity to any of the foregoing.

For example, the antibody comprises the CH1 and/or CL portion of IgG4 in Table 5, a conservative variant thereof or a sequence with at least 50%, 60%, 70%, 89%, 90% or 95% sequence identity thereto. In a further embodiment, the antibody comprises sequences in Table 5 or a conservative variant thereof or a sequence with at least 50%, 60%, 70%, 89%, 90% or 95% sequence identity thereto.

For example, the antibody comprises a KD of at least or about $1 \times 10^{-10}$, at least or about $8 \times 10^{-11}$, at least or about $6 \times 10^{-11}$, at least or about $4 \times 10^{-11}$ or at least or about $2 \times 10^{-11}$ for the cyclic peptide with sequence of SEQ ID NO: 12.

Human antibodies specific to a particular antigen may be identified by a phage display strategy (Jespers et al. Bio/Technology, 12: 899-903, 1994). In one approach, the heavy chain of a rodent antibody directed against a specific antigen is cloned and paired with a repertoire of human light chains for display as Fab fragments on filamentous phage. The phage is selected by binding to antigen. The selected human light chain is subsequently paired with a repertoire of human heavy chains for display on phage, and the phage is again selected by binding to antigen. The result is a human antibody Fab fragment specific to a particular antigen. In another approach, libraries of phage are produced where members display different human antibody fragments (Fab or Fv) on their outer surfaces (Dower et al., WO 91/17271 and McCafferty et al., WO 92/01047). Phage displaying antibodies with a desired specificity are selected by affinity enrichment to a specific antigen. The human Fab or Fv fragment identified from either approach may be recloned for expression as a human antibody in mammalian cells.

Human antibodies are optionally obtained from transgenic animals (US Patent Nos. 6,150,584; 6,114,598; and 5,770,429). In this approach the heavy chain joining region (JH) gene in a chimeric or germ-line mutant mouse is deleted. Human germ-line immunoglobulin gene array is subsequently transferred to such mutant mice. The resulting transgenic mouse is then capable of generating a full repertoire of human antibodies upon antigen challenge.

Humanized antibodies are typically produced as antigen binding fragments such as Fab, Fab' F(ab')2, Fd, Fv and single domain antibody fragments, or as single chain antibodies in which the heavy and light chains are linked by a spacer. Also, the human or humanized antibodies may exist in monomeric or polymeric form. The humanized antibody optionally comprises one non-human chain and one humanized chain (i.e. one humanized heavy or light chain).

Antibodies including humanized or human antibodies are selected from any class of immunoglobulins including: IgM, IgG, IgD, IgA or IgE; and any isotype, including: IgG1, IgG2, IgG3 and IgG4. The humanized or human antibody may include sequences from one or more than one isotype or class.

Additionally, antibodies specific for the epitopes described herein are readily isolated by screening antibody phage display libraries. For example, an antibody phage library is optionally screened by using a disease specific epitope of the current invention to identify antibody fragments specific for the disease specific epitope. Antibody fragments identified are optionally used to produce a variety of recombinant antibodies that are useful with different embodiments of the present invention. Antibody phage display libraries are commercially available, for example, through Xoma (Berkeley, California) Methods for screening antibody phage libraries are well known in the art.

In a preferred embodiment of the invention the antibodies herein disclosed can be single chain antibodies. The humanized antibodies described are also in an embodiment, single chain antibodies.

The disclosure also relates to antibodies that compete for binding to a cyclic peptide having sequence of SEQ ID NO: 12, and/or human A-beta oligomers with an antibody comprising the CDR sequences as recited in Table 2, or comprising a sequence as provided in any one of Tables 3, 4A, 4B and 8.

Competition between antibodies can be determined for example using an assay in which an antibody under test is assessed for its ability to inhibit specific binding of a reference antibody to the common antigen. A test antibody competes with a reference antibody if an excess of a test antibody (e.g., at least a 2 fold, 5, fold, 10 fold or 20 fold) inhibits binding of the reference antibody by at least 50%, at least 75%, at least 80%, at least 90% or at least 95% as measured in a competitive binding assay.

For example the antibody is isolated. In an embodiment, the antibody is an exogenous antibody.

For example, the antibody does not bind monomeric A-beta, for example under conditions described in the Examples. In an embodiment, the antibody does not bind A-beta in senile plaques, for example in situ in AD brain tissue, for example under conditions described in the Examples.

For example, the antibody does not selectively bind monomeric A-beta compared to native- or synthetic- oligomeric A-beta.

For example, the A-beta oligomer comprises A-beta 1-42 subunits.

For example, the antibody lacks A-beta fibril plaque (also referred to as senile plaque) staining, for example as measured by immuohistochemistry. Absence of plaque staining can be assessed by comparing to a positive control such as A-beta-specific antibodies 6E10 and 4G8 (Biolegend, San Diego, CA), or 2C8 (Enzo Life Sciences Inc., Farmingdale, NY) and an isotype control. An antibody described herein lacks or has negligible A-beta fibril plaque staining if the antibody does not show typical plaque morphology staining and the level of staining is comparable to or no more than 2 fold the level seen with an IgG negative isotype control. The scale can for example set the level of staining with isotype control at 1 and with 6E10 at 10. An antibody lacks A-beta fibril plaque staining if the level of staining on such a scale is 2 or less. In embodiment, the antibody shows minimal A-beta fibril plaque staining, for example on the foregoing scale, levels scored at less about or less than 3.

A further aspect is an antibody according to the invention conjugated to a detectable label or cytotoxic agent. In an embodiment, the detectable label is a positron-emitting radionuclide. A positron-emitting radionuclide can be used for example in PET imaging.

The disclosure also relates to an antibody complex comprising an antibody described herein and/or a binding fragment thereof and oligomeric A-beta.

A further aspect is an isolated nucleic acid encoding an antibody described herein.

Nucleic acids encoding a heavy chain or a light chain or parts thereof are also provided, for example encoding variable chains described herein and codon optimized and codon degenerate versions thereof.

The present disclosure also relates to variants of the nucleic acid sequences that encode for the antibody and/or binding fragment thereof disclosed herein. For example, the variants include nucleotide sequences that hybridize to the nucleic acid sequences encoding the antibody and/or binding fragment thereof disclosed herein under at least moderately stringent hybridization conditions or codon degenerate or optimized sequences In another embodiment, the variant nucleic acid sequences have at least 50%, at least 60%, at least 70%, most preferably at least 80%, even more preferably at least 90% and even most preferably at least 95% sequence identity to nucleic acid sequences encoding any of the variable chains described herein.

A further aspect is an isolated nucleic acid encoding an antibody described herein, for example the nucleic acids shown in SEQ ID NOs: 45 and 65.

In a preferred embodiment said nucleic acid is comprised in a vector. For example, the vector is an isolated vector.

The vector can be any vector, including vectors suitable for producing an antibody and/or binding fragment thereof or expressing a peptide sequence described herein.

The nucleic acid molecules may be incorporated in a known manner into an appropriate expression vector which ensures expression of the protein. Possible expression vectors include but are not limited to cosmids, plasmids, or modified viruses (e.g. replication defective retroviruses, adenoviruses and adeno-associated viruses). The vector should be compatible with the host cell used. The expression vectors are "suitable for transformation of a host cell", which means that the expression vectors contain a nucleic acid molecule encoding the peptides corresponding to epitopes or antibodies described herein.

For example, the vector is suitable for expressing for example single chain antibodies by gene therapy. The vector can be adapted for specific expression in neural tissue, for example using neural specific promoters and the like. In an embodiment, the vector comprises an IRES and allows for expression of a light chain variable region and a heavy chain variable region. Such vectors can be used to deliver antibody in vivo.

Suitable regulatory sequences may be derived from a variety of sources, including bacterial, fungal, viral, mammalian, or insect genes.

Examples of such regulatory sequences include: a transcriptional promoter and enhancer or RNA polymerase binding sequence, a ribosomal binding sequence, including a translation initiation signal. Additionally, depending on the host cell chosen and the vector employed, other sequences, such as an origin of replication, additional DNA restriction sites, enhancers, and sequences conferring inducibility of transcription may be incorporated into the expression vector.

For example, the regulatory sequences direct or increase expression in neural tissue and/or cells.

For example, the vector is a viral vector.

The recombinant expression vectors may also contain a marker gene which facilitates the selection of host cells transformed, infected or transfected with a vector for expressing an antibody or epitope peptide described herein.

The recombinant expression vectors may also contain expression cassettes which encode a fusion moiety (i.e. a "fusion protein") which provides increased expression or stability of the recombinant peptide; increased solubility of the recombinant peptide; and aid in the purification of the target recombinant peptide by acting as a ligand in affinity purification, including for example tags and labels described herein. Further, a proteolytic cleavage site may be added to the target recombinant protein to allow separation of the recombinant protein from the fusion moiety subsequent to purification of the fusion protein. Typical fusion expression vectors include pGEX (Amrad Corp., Melbourne, Australia), pMAL (New England Biolabs, Beverly, MA) and pRIT5 (Pharmacia, Piscataway, NJ) which fuse glutathione S-transferase (GST), maltose E binding protein, or protein A, respectively, to the recombinant protein.

Systems for the transfer of genes for example into neurons and neural tissue both in vitro and in vivo include vectors based on viruses, most notably Herpes Simplex Virus, Adenovirus, Adeno-associated virus (AAV) and retroviruses including lentiviruses. Alternative approaches for gene delivery include the use of naked, plasmid DNA as well as liposome-DNA complexes. Another approach is the use of AAV plasmids in which the DNA is polycation-condensed and lipid entrapped and introduced into the brain by intracerebral gene delivery (Leone et al. US Application No. 2002076394).

Accordingly, the disclosure also relates to the compounds, immunogens, nucleic acids, vectors and antibodies described herein formulated in vesicles such as liposomes, nanoparticles, and viral protein particles, for example for delivery of antibodies, compounds, immunogens and nucleic acids described herein. In particular synthetic polymer vesicles, including polymersomes, can be used to administer antibodies.

Also provided in another aspect is a cell, optionally an isolated and/or recombinant cell, expressing an antibody described herein.

The recombinant cell can be generated using any cell suitable for producing a polypeptide, for example suitable for producing an antibody and/or binding fragment thereof. For example to introduce a nucleic acid (e.g. a vector) into a cell, the cell may be transfected, transformed or infected, depending upon the vector employed.

Suitable host cells include a wide variety of prokaryotic and eukaryotic host cells. For example, the proteins described herein may be expressed in bacterial cells such as E. coli, insect cells (using baculovirus), yeast cells or mammalian cells.

For example, the cell is a eukaryotic cell selected from a yeast, plant, worm, insect, avian, fish, reptile and mammalian cell.

For example, the mammalian cell is a myeloma cell, a spleen cell, or a hybridoma cell.

For example, the cell is a neural cell.

Yeast and fungi host cells suitable for expressing an antibody or peptide include, but are not limited to Saccharomyces cerevisiae, Schizosaccharomyces pombe, the genera Pichia or Kluyveromyces and various species of the genus Aspergillus. Examples of vectors for expression in yeast S. cerivisiae include pYepSec1, pMFa, pJRY88, and pYES2 (Invitrogen Corporation, San Diego, CA). Protocols for the transformation of yeast and fungi are well known to those of ordinary skill in the art.

Mammalian cells that may be suitable include, among others: COS (e.g., ATCC No. CRL 1650 or 1651), BHK (e.g. ATCC No. CRL 6281), CHO (ATCC No. CCL 61), HeLa (e.g., ATCC No. CCL 2), 293 (ATCC No. 1573) and NS-1 cells. Suitable expression vectors for directing expression in mammalian cells generally include a promoter (e.g., derived from viral material such as polyoma, Adenovirus 2, cytomegalovirus and Simian Virus 40), as well as other transcriptional and translational control sequences. Examples of mammalian expression vectors include pCDM8 and pMT2PC.

For example, the cell is a fused cell such as a hybridoma cell, the hybridoma cell producing an antibody specific and/or selective for an epitope or epitope sequence described herein, including for example that selectively binds A-beta oligomers over A-beta monomers, selectively binds an epitope sequence presented in a cyclic compound relative to a linear compound or lacks or has negligible plaque binding.

### III. Compositions

A further aspect is a composition comprising the antibody described herein or the immunoconjugate according to the invention and a diluent.

Suitable diluents for nucleic acids include but are not limited to water, saline solutions and ethanol.

Suitable diluents for polypeptides, including antibodies or fragments thereof and/or cells include but are not limited to saline solutions, pH buffered solutions and glycerol solutions or other solutions suitable for freezing polypeptides and/or cells.

For example, the composition is a pharmaceutical composition comprising any of the antibodies, nucleic acids or vectors disclosed herein, and optionally comprising a pharmaceutically acceptable carrier.

The compositions described herein can be prepared by per se known methods for the preparation of pharmaceutically acceptable compositions that can be administered to subjects, optionally as a vaccine, such that an effective quantity of the active substance is combined in a mixture with a pharmaceutically acceptable vehicle.

Pharmaceutical compositions include, without limitation, lyophilized powders or aqueous or non-aqueous sterile injectable solutions or suspensions, which may further contain antioxidants, buffers, bacteriostats and solutes that render the compositions substantially compatible with the tissues or the blood of an intended recipient. Other components that may be present in such compositions include water, surfactants (such as Tween), alcohols, polyols, glycerin and vegetable oils, for example. Extemporaneous injection solutions and suspensions may be prepared from sterile powders, granules, tablets, or concentrated solutions or suspensions. The composition may be supplied, for example but not by way of limitation, as a lyophilized powder which is reconstituted with sterile water or saline prior to administration to the patient.

Pharmaceutical compositions may comprise a pharmaceutically acceptable carrier. Suitable pharmaceutically acceptable carriers include essentially chemically inert and nontoxic compositions that do not interfere with the effectiveness of the biological activity of the pharmaceutical composition. Examples of suitable pharmaceutical carriers include, but are not limited to, water, saline solutions, glycerol solutions, ethanol, N-(1(2,3-dioleyloxy)propyl)N,N,N-trimethylammonium chloride (DOTMA), diolesylphosphotidyl-ethanolamine (DOPE), and liposomes. Such compositions should contain a therapeutically effective amount of the compound, together with a suitable amount of carrier so as to provide the form for direct administration to the patient.

The composition may be in the form of a pharmaceutically acceptable salt which includes, without limitation, those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylarnino ethanol,

In an embodiment, the composition comprises an antibody described herein. For example, the composition comprises an antibody described herein and a diluent. For example, the composition is a sterile composition.

The disclosure also relates to an antibody complex comprising an antibody described herein and A-beta, optionally A-beta oligomer. The complex may be in solution or comprised in a tissue, optionally in vitro.

### IV. Kits

The disclosure also relates to a kit comprising i) an antibody and/or binding fragment thereof, ii) a nucleic acid of said antibody or a part thereof, iii) composition comprising an antibody, nucleic acid or cell described herein or iv) a recombinant cell described herein, comprised in a vial such as a sterile vial or other housing and optionally a reference agent and/or instructions for use thereof.

For example, the kit further comprises one or more of a collection vial, standard buffer and detection reagent.

For example, the kit is for diagnosing or monitoring Alzheimer's disease or a condition involving oligomeric Abeta.

### V. Methods

The disclosure also relates to methods for making the antibodies described herein.

In particular, methods of making an antibody an antibody described herein selective for a conformational epitope of HHQK (SEQ ID NO: 7) using an antibody described herein are disclosed, the method comprising administering to a subject, optionally a non-human subject, a cyclic compound comprising an epitope sequence described herein, and isolating antibody producing cells or antibodies that comprise the CDRs described herein.

For example, the method is for making a monoclonal antibody using for example a method as described herein.

For example, a method of making a chimeric antibody or binding fragment thereof is described, the method comprising using recombinant technology to subcloning a nucleic acid encoding the variable region of an antibody (heavy and/or light) described herein into a vector comprising a nucleic acid encoding a human antibody constant domain (e.g. IgG1, 2, 3, or 4), optionally with or without the Fc portion to produce a chimeric antibody vector; and expressing the chimeric antibody vector in a cell; and isolating the antibody. In an embodiment, the chimera is a mouse human chimera.

For example, the method is for making a humanized antibody using for example a method described herein. For example the method comprises making a chimeric intermediate. The variable regions of the chimeric intermediate are for example mutagenized to introduce one or more amino acid changes outside the CDR regions. For example, one or more CDR coding sequences described herein are inserted into a human antibody scaffold.

Antibodies produced using a cyclic compound are selected as described herein and in the Examples such. In an embodiment, the method comprises isolating antibodies that specifically or selectively bind cyclic peptide over linear peptide, are specific for the epitope sequence, specifically bind oligomer and/or lack or negligibly bind plaque in situ and/or corresponding linear peptide, optionally using a method described herein.

We disclose the use of the antibody or immunoconjugate for assessing whether a biological sample comprises A-beta the method comprising contacting the biological sample with an antibody described herein and/or detecting the presence of any antibody complex. In an embodiment, the method is for detecting whether a biological sample comprises oligomeric A-beta.

For example, the method comprises:

a. contacting the biologic sample with an antibody described herein that is specific and/or selective for A-beta oligomer herein under conditions permissive to produce an antibody: A-beta oligomer complex; and

b. detecting the presence of any complex;

wherein the presence of detectable complex is indicative that the sample may contain A-beta oligomer.

For example, the level of complex formed is compared to a test antibody such as a suitable Ig control or irrelevant antibody.

For example, the detection is quantitated and the amount of complex produced is measured. The measurement can for example be relative to a standard.

For example, the measured amount is compared to a control.

For example, the method comprises:

(a) contacting a test sample of said subject with an antibody described herein, under conditions permissive to produce an antibody-antigen complex;

(b) measuring the amount of the antibody-antigen complex in the test sample; and

(c) comparing the amount of antibody-antigen complex in the test sample to a control;

wherein detecting antibody-antigen complex in the test sample as compared to the control indicates that the sample comprises A-beta.

The control can be a sample control (e.g. from a subject without AD, or from a subject with a particular form of AD, mild, moderate or advanced), or be a previous sample from the same subject for monitoring changes in A-beta oligomer levels in the subject. Alternatively, the control can be a value derived from a plurality of patients with or without AD.

For example, the antibody is an antibody having the CDR sequences described herein. Preferably, the antibody is a humanized antibody. In an embodiment, the antibody is a chimeric antibody.

For example, the sample is a biological sample. Preferably, the sample comprises brain tissue or an extract thereof and/or CSF. In an embodiment, the sample comprises whole blood, plasma or serum. For example, the sample is obtained from a human subject. In an embodiment, the subject is suspected of, at a risk of or has AD.

A number of methods can be used to detect an A-beta: antibody complex and thereby determine A-beta oligomers is present in a sample using the antibodies described herein, including immunoassays such as flow cytometry, Western blots, ELISA, SPR and immunoprecipitation followed by SDS-PAGE immunocytochemistry.

As described in the Examples surface plasmon resonance technology can be used to assess conformation specific binding. If the antibody is labeled or a detectably labeled secondary antibody specific for the complex antibody is used, the label can be detected. Commonly used reagents include fluorescent emitting and HRP labeled antibodies. In quantitative methods, the amount of signal produced can be measured by comparison to a standard or control. The measurement can also be relative.

A further aspect includes the immunoconjugate described herein for use in measuring a level of A-beta in a subject at risk or suspected of having or having Alzheimer's disease, wherein the antibody is conjugated to a detectable label, preferably wherein the label is a positron emitting radionuclide., optionally where the A-beta to be measured or imaged is oligomeric A-beta. For example, the method comprises administering to a subject at risk or suspected of having or having AD, an antibody described herein conjugated to a detectable label; and detecting the label, optionally quantitatively detecting the label. The label in an embodiment is a positron emitting radionuclide which can for example be used in PET imaging.

The methods may also be combined with other tests for AD or cognitive impairment. For example, synaptic protein levels, such as SNAP-25 or synaptic vesicle glycoprotein 2a (SVG2a) (Sci Transl Med. 2016 Jul 20;8(348):348ra96. doi: 10.1126/scitranslmed.aaf6667) in CSF can be measured. For example, flourodeoxyglucose PET (FDG-PET) is used as an indirect measure of synaptic metabolism.

Detecting A-beta levels using an antibody described herein can be used alone or in combination with other methods to monitor response to treatment.

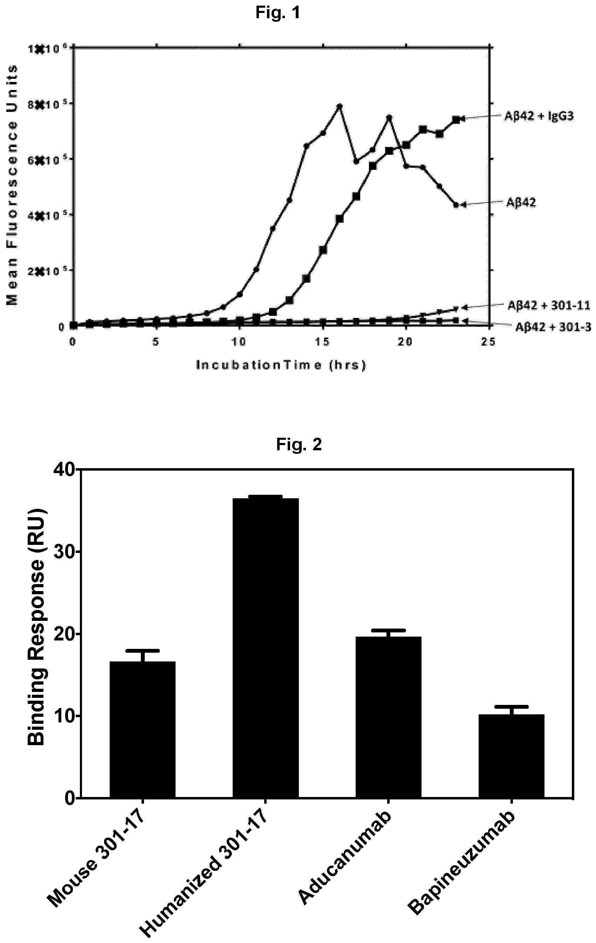

It is demonstrated herein that antibodies raised against cyclo(CGHHQKG) (SEQ ID NO: 12), comprising the CDR sets described herein can specifically and/or selectively bind A-beta oligomers. Oligomeric A-beta species are believed to be the toxic propagating species in AD. Further as shown in FIG. 1 and described in the Examples, these antibodies are specific for oligomers, inhibited A-beta aggregation and A-beta oligomer propagation. Accordingly, also described are methods of inhibiting A-beta oligomer propagation, the method comprising contacting a cell or tissue expressing A-beta with or administering to a subject in need thereof an effective amount of an A-beta oligomer specific or selective antibody described herein to inhibit A-beta aggregation and/or oligomer propagation. In vitro the assay can be monitored as described in the Examples.

In an aspect, the antibodies are for use in the treatment of AD and/or other A-beta amyloid related diseases, in particular Lewy Body dementia and/or cerebral amyloid angiopathy. Variants of Lewy body dementia and in inclusion body myositis (a muscle disease) exhibit similar plaques as AD and A-beta can also form aggregates implicated in cerebral amyloid angiopathy. As mentioned, the antibodies comprising the CDR sets as well as when in the humanized antibodies sequences described herein bind oligomeric A-beta which is believed to be a toxigenic species of A-beta in AD and inhibit formation of toxigenic A-beta oligomers in vitro.

We disclose antibody or immunoconjugate for use in treating AD and/or other A-beta amyloid related diseases, the method comprising administering to a subject in need thereof an effective amount of an antibody described herein comprising a CDR set described herein, optionally a humanized antibody described in Table 4A or 4B or selective or a pharmaceutical composition comprising said antibody, to a subject in need thereof. In other embodiments, nucleic acids encoding the antibodies described herein can also be administered to the subject, optionally using vectors suitable for delivering nucleic acids in a subject.

According to an aspect of the invention there is provided an effective amount of an antibody or immunoconjugate according to the invention, or a pharmaceutical composition comprising said antibody or immunoconjugate, for use in treating Alzheimer's disease, Lewy body dementia and/or cerebral amyloid angiopathy in a subject in need thereof, preferably wherein the antibody or immunoconjugate is for administration directly to the brain or other portion of the CNS.In an embodiment, a biological sample from the subject to be treated is assessed for the presence or levels of A-beta using an antibody described herein. In an embodiment, a subject with detectable A-beta levels (e.g. A-beta antibody complexes measured in vitro or measured by imaging) is treated with the antibody.

The antibody, peptides and nucleic acids can for example be comprised in a pharmaceutical composition as described herein, and formulated for example in vesicles for improving delivery.

One or more antibodies targeting HHQK (SEQ ID NO: 7) can be administered in combination. In addition the antibodies disclosed herein can be administered with one or more other treatments such as a beta-secretase inhibitor or a cholinesterase inhibitor.

Also provided are uses of the compositions, antibodies, isolated peptides, and nucleic acids for treating AD or A-beta amyloid related diseases.

The compositions, antibodies, isolated peptides and nucleic acids, vectors etc. described herein can be administered for example, by parenteral, intravenous, subcutaneous, intramuscular, intracranial, intraventricular, intrathecal, intraorbital, ophthalmic, intraspinal, intracisternal, intraperitoneal, intranasal, aerosol or oral administration.

For example, the pharmaceutical composition is administered systemically.

For example, the pharmaceutical composition is administered directly to the brain or other portion of the CNS. For example such methods include the use of an implantable catheter and a pump, which would serve to discharge a pre-determined dose through the catheter to the infusion site. A person skilled in the art would further recognize that the catheter may be implanted by surgical techniques that permit visualization of the catheter so as to position the catheter adjacent to the desired site of administration or infusion in the brain. Such techniques are described in Elsberry et al. U.S. Patent 5,814,014 "Techniques of Treating Neurodegenerative Disorders by Brain Infusion". Also contemplated are methods such as those described in US patent application 20060129126 (Kaplitt and During "Infusion device and method for infusing material into the brain of a patient". Devices for delivering drugs to the brain and other parts of the CNS are commercially available (eg. SynchroMed® EL Infusion System; Medtronic, Minneapolis, Minnesota).

For example, the pharmaceutical composition is administered to the brain using methods such as modifying the compounds to be administered to allow receptor-mediated transport across the blood brain barrier.

Further examples contemplate the co-administration of the compositions, antibodies, isolated peptides and nucleic acids described herein with biologically active molecules known to facilitate the transport across the blood brain barrier.

Also contemplated, are methods for administering the compositions, antibodies, isolated peptides, and nucleic acids described herein across the blood brain barrier such as those directed at transiently increasing the permeability of the blood brain barrier as described in US patent 7012061 "Method for increasing the permeability of the blood brain barrier".

The above disclosure generally describes the present application. A more complete understanding can be obtained by reference to the following specific examples. These examples are described solely for the purpose of illustration and are not intended to limit the scope of the application. Changes in form and substitution of equivalents are contemplated as circumstances might suggest or render expedient. Although specific terms have been employed herein, such terms are intended in a descriptive sense and not for purposes of limitation.

The following non-limiting examples are illustrative of the present disclosure:

## Examples

## Example 1

## Antibody Generation

## Methods and Materials

### Immunogen

Cyclo(CGHHQKG) (SEQ ID NO:12) peptide was generated at CPC Scientific, Sunnyvale, CA, USA (both cyclic and linear), and conjugated to KLH (for immunizing) and BSA (for screening) using a trifluoroacetate counter ion protocol. A linear peptide of the same sequences, CGHHQKG (SEQ ID NO: 12), were also made.

### Antibodies

Hybridomas and monoclonal antibodies were generated to cyclo(CGHHQKG) (SEQ ID NO: 12) linked to Keyhole Limpet Hemocyanin (KLH).

### Fusion 1 Hybridoma Development

Lymphocytes were isolated and fused with murine SP2/0 myeloma cells in the presence of poly-ethylene glycol (PEG 1500). Fused cells were cultured using HAT selection. T.

### Hybridoma Analysis

Tissue culture supernatants from the hybridomas were tested by indirect ELISA on screening antigen (cyclic peptide-BSA) and probed for both IgG and IgM antibodies using a Goat anti-IgG/IgM(H&L)-HRP secondary and developed with TMB substrate. Positive cultures were retested on screening antigen to confirm secretion and on an irrelevant antigen (Human Transferrin) to eliminate non-specific mAbs and rule out false positives. Selected clones were isotyped by antibody trapping ELISA to determine if they are IgG or IgM isotype. Selected clones were also tested by indirect ELISA on other cyclic peptide-BSA conjugates as well as linear peptide-BSA conjugates to evaluate cross-reactivity and linker reactivity. Antibodies were also screened by SPR analysis.

### ELISA Antibody Screening

ELISA plates were coated with 1) 0.1ug/well cyclopeptide-conjugated -BSA at 100uL/well in carbonate coating buffer (pH 9.6) O/N at 4C; 2) 0.1ug/well linear -peptide-conjugated - BSA at 100uL/well in carbonate coating buffer (pH 9.6) O/N at 4C; or 3) 0.1ug/well Negative-Peptide at 100uL/well in carbonate coating buffer (pH 9.6) O/N at 4C. Primary Antibody: Hybridoma supernatant at 100 uL/well incubated for 1 hour at 37C with shaking. Secondary Antibody 1:10,000 Goat anti-mouse IgG/IgM(H+L)-HRP at 100uL/well in PBS-Tween for 1 hour at 37C with shaking. All washing steps were performed for 30 mins with PBS-Tween. The substrate TMB was added at 50uL/well, developed in the dark and stopped with equal volume 1M HCl.

*SPR Binding Assays*

**SPR analysis of Antibody binding to cyclic peptides, A-beta monomers and oligomers**

**A-beta Monomer and Oligomer Preparation:**

Recombinant A-beta40 and 42 peptides (California Peptide, Salt Lake City UT, USA) were dissolved in ice-cold hexafluoroisopropanol (HFIP). The HFIP was removed by evaporation overnight and dried in a SpeedVac centrifuge. To prepare monomers, the peptide film was reconstituted in DMSO to 5mM, diluted further to 100µM in dH2O and used immediately. Oligomers were prepared by diluting the 5mM DMSO peptide solution in phenol red-free F12 medium (Life Technologies Inc., Burlington ON, Canada) to a final concentration of 100µM and incubated for 24 hours to 7 days at 4°C.

**SPR Analysis of Cyclic Peptide, A-beta Monomer and Oligomer binding:**

All SPR measurements were performed using a Molecular Affinity Screening System (MASS-1) (Sierra Sensors GmbH, Hamburg, Germany), an analytical biosensor that employs high intensity laser light and high speed optical scanning to monitor binding interactions in real time. The primary screening of tissue culture supernatants was performed using an SPR direct binding assay, whereby BSA-conjugated peptides, A-beta42 Monomer and A-beta42 Oligomer are covalently immobilized on individual flow cells of a High Amine Capacity (HAC) sensorchip (Sierra Sensors GmbH, Hamburg, Germany) and antibodies flowed over the surface. Each sample was diluted and injected in duplicate over the immobilized peptide and BSA reference surfaces, followed by injection of running buffer only for the dissociation phase. After every analytical cycle, the sensor chip surfaces were regenerated. Sensorgrams were double-referenced by subtracting out binding from the BSA reference surfaces and blank running buffer injections, and binding response report points collected in the dissociation phase.

Protein G purified mAbs were analyzed in a secondary screen using an SPR indirect (capture) binding assay, whereby the antibodies were immobilized on a protein A-derivatized sensorchip (XanTec Bioanalytics GmbH, Duesseldorf, Germany) and A-beta40 Monomer, A-beta42 Oligomer, pooled soluble brain extracts flowed over the surface. The specificity of the antibodies was verified in an SPR direct binding assay by covalently immobilizing A-beta42 Monomer and A-beta42 Oligomer on individual flow cells of a HAC sensorchip and flowing purified mAbs over the surface.

**Antibody Sequencing**

The CDR and variable regions of the heavy and light chains were sequenced. Immunoglobulin gene transcripts expressed by the hybridomas were amplified from cDNA generated from the hybridoma cells using standard RT-PCR and sequenced using a standard dye-terminator capillary sequencing method.

**Humanized Antibodies**

Humanized IgG4 antibody constructs were prepared for 301-17 and sequenced (Abzena Cambridge UK).

Briefly RNA was extracted from the hybridoma 301-17 cell pellet using an RNeasy Mini Kit (Qiagen, Hilden, Germany). V-regions were amplified by RT-PCR using degenerate primer pools for murine antibody signal sequences together with constant region primers for each of IgG and Igκ. Heavy chain V region mRNA was amplified using a set of six degenerate primer pools (A to F) specific for VH signal sequences together with IgG-specific constant region primers. The light chain V region mRNA was amplified using a set of eight signal sequence-specific degenerate primer pools, seven for the kappa cluster (Igκ-A to Igκ-G) and one for the lambda cluster (IgA), together with κ or A constant region primers. The PCR products obtained were purified, cloned into a 'TA' cloning vector (pGEM-T Easy, Promega, Madison, USA), transformed into *E. coli* and individual colonies sequenced.

Chimeric constructs (V0H0 and V0k0) were prepared using the variable regions from the hybridoma which were cloned into a human IgG4 framework. The chimeric constructs were then humanized to create 6 humanized heavy chains (VH1-6) and 6 light chains (Vk1-6). VH1-6 and Vk4-6 constructs were mixed to create different humanized antibodies e.g. VH2Vk4.

The S241P hinge variant comprises an altered disulfide bond arrangement of an IgG4 molecule by mutation of the Cys at the N terminus of the heavy chain constant domain 1 ($C_H1$) (Kabat position 127) to a Ser and introduction of a Cys at a variety of positions (positions 227-230) at the C terminus of $C_H1$. An inter-LC-$C_H1$ disulfide bond is formed [17].

The fully humanized antibodies were prepared using Composite Human Antibody™ technology. The humanized variable region genes were cloned into vectors encoding a human IgG4 (S241P hinge variant) heavy chain constant domain and a human kappa light chain constant domain. Chimeric and humanized antibodies were transiently expressed in CHO cells and Protein A purified and tested. All 301-17 humanized antibodies selectively bound the cyclic peptide conjugated to BSA with binding affinities within 2-fold of the reference chimeric antibody and greater than 10 fold and about 20 of the reference monoclonal antibody. The chimeric and humanized antibodies had a KD(M) of about $2 \times 10^{-11}$ whereas the KD of the monoclonal antibody had a KD (M) of $4 \times 10^{-10}$ for the cyclic peptide. As shown in Fig. 2 this translates to an improved affinity for oligomeric Abeta.

Binding was determined using single cycle Biacore analysis. Antibodies were analysed in two separate experiments.

Humanized antibody sequences are provided in Table 4B (301-17). The CDR sequences of each antibody sequences are bolded and underlined. The CDRs of 301-11 or any other antibody described herein can be used to replace the CDRs in the humanized constructs as shown for example in Table 4A.

## Results

ELISA testing found that hybridoma clones bound the cyclopeptide preferentially over the linear peptide. Clones 301-3, 301-11 and 301-17 raised against cyclo(CGHHQKG) were selected for further analysis.

Isotyping revealed 301-3, 301-11 and 301-17 were IgG3 subtypes.

Antibodies were tested in one or more assays for their ability to bind cyclic peptide, linear peptide, A-beta 1-42 monomer and A-beta 1-42 oligomers prepared as described above.

ELISA and SPR assays confirmed that the clones preferentially bound the cylopeptide relative to the linear peptide (and were not cross reactive to unrelated cyclic peptides) and/or preferentially bound Aβ oligomers relative to monomers. The results of the binding analysis using SPR with hybridoma culture supernatants are shown in Table 1A.

Antibodies purified from the hybridoma supernatants were immobilized and assayed for their ability to bind Abeta oligomers by SPR. The results are shown in Table 1B.

### Table 1A

|  | Cyclic Peptide(RU) | Linear Peptide(RU) | Ab42 Monomer(RU) | Ab42 Oligomer(RU) |
|---|---|---|---|---|
| 301-11 | 488 | 210.5 | 21.6 | 75.3 |
| 301-3 | 468.9 | 60.6 | -1.8 | 56.8 |

### Table 1B

|  | Ab42 Monomer (RU) | Ab42 Oligomer(RU) |
|---|---|---|
| 301-3 | -23.8 | 15.5 |
| 301-11 | -14.1 | -2.8 |
| 301-17 | -27.1 | 147.8 |

## Antibody Sequence

Clones 301-3, 301-11 and 301-17 antibodies were sequenced. The CDR sequences of 301-3 and 301-11 are provided in Table 2. The CDRs for 301-17 are provided in SEQ ID Nos 74-79. The consensus DNA sequence and polypeptide sequences of the variable portion of the heavy and light chain of the antibodies are provided in Table 3.

As shown in Table 2, the heavy chain CDRs for 301-3 and 301-11 were identical for CDRs 1 and 2 and CDR3 varied at one position.

Two light chains were sequenced. One light chain was near identical to the light chain for 301-11.

Humanized antibodies were prepared for 301-17 and sequenced (Abzena Cambridge UK). Humanized antibody sequences are provided in Table 4A (301-11) and 4B (301-17). The CDR sequences of each antibody sequences are bolded and underlined.

### Table 2

| Antibody | Chain | CDR | Sequence | SEQ ID NO. |
|---|---|---|---|---|
| 301-11 | Heavy | CDR-H1 | GFTFSDYY | 1 |
| 301-11 |  | CDR-H2 | ISDGGSYT | 2 |
| 301-11 |  | CDR-H3 | ARDYYGSSSYTSGFAY | 3 |

(continued)

| Antibody | Chain | CDR | Sequence | SEQ ID NO. |
|---|---|---|---|---|
| 301-11 | Light | CDR-L1 | QSLLNSRTRKNY | 4 |
| 301-11 | | CDR-L2 | WAS | 5 |
| 301-11 | | CDR-L3 | KQSYNLYT | 6 |
| 301-03-1 | Heavy | CDR-H1 | GFTFSDYY | 1 |
| 301-03-1 | | CDR-H2 | ISDGGSYT | 2 |
| 301-03 -1 | | CDR-H3 | ARDYYGSNSYTSGFAY | 80 |
| 301-03-1 | Light | CDR-L1 | QSLLNSRTRKNY | 4 |
| 301-03-1 | | CDR-L2 | WAS | 5 |
| 301-03-1 | | CDR-L3 | KQSYNLYT | 6 |
| 301-03-2 | Heavy | CDR-H1 | GFTFSDYY | 1 |
| 301-03-2 | | CDR-H2 | ISDGGSYT | 2 |
| 301-03-2 | | CDR-H3 | ARDYYGSNSYTSGFAY | 80 |
| 301-03-2 | Light | CDR-L1 | QSIVHSNGNTY | 81 |
| 301-03-2 | | CDR-L2 | KVS | 82 |
| 301-03-2 | | CDR-L3 | FQGSHVPLT | 83 |
| 301-17 | Heavy | CDR-H1 | GYSFTSYW | 74 |
| 301-17 | | CDR-H2 | VHPGRGVST | 75 |
| 301-17 | | CDR-H3 | SRSHGNTYWFFDV | 76 |
| 301-17 | Light | CDR-L1 | QSIVHSNGNTY | 77 |
| 301-17 | | CDR-L2 | KVS | 78 |
| 301-17 | | CDR-L3 | FQGSHVPFT | 79 |

**Table 3=**

Consensus DNA sequence and translated protein sequences of the variable region. The complementarity determining regions (CDRs) are underlined according to IMTG/LIGM-DB.

| Antibody and Isotype | Consensus cDNA Sequence | Polypeptide sequence |
|---|---|---|
| 301-11 IgG3 SEQ ID NO: 8, 9 | ATGAACTTTGGGCTCAGCTTGATTTTCCTTGTCCTTGTTTTTAAAA GGTGTCCAGTGTGAAGTGCAGCTGGTGGAGTCTGGGGGAGGCTTA GTGAAGCCTGGAGGGTCCCTGAAACTCTCCTGTGCAGCCTCT**GGA TTCACTTTCAGTGACTATTAC**ATGTATTGGGTTCGCCAGACTC CGGAAAAGAGGCTGGAGTGGGTCGCAACC**ATTAGTGATGGTGG TAGTTACACC**TCCTATCCAGACAGTGTGAAGGGACGATTCACCA TCTCCAGAGACAATGCCAAGAACAACCTGTACCTGCAAATGAGCA GTCTGAGGTCTGAGGACACAGCCATGTATTACTGT**GCAAGAGAT TACTACGGTAGTAGTAGCTACACCTCGGGCTTTGCTTAC**TG GGGCCAAGGGACTCTGGTCACTGTCTCTGCA | MNFGLSLIFLVLVLKG VQCEVQLVESGGGLVK PGGSLKLSCAAS**GFTF SDYY**MYWVRQTPEKRL EWVAT**ISDGGSYT**SY PDSVKGRFTISRDNAK NNLYLQMSSLRSEDTA MYYC**ARDYYGSSSYT SGFAY**WGQGTLVTVSA |
| 301-11 Kappa SEQ ID NO: 10,11 | ATGGATTCACAGGCCCAGGTTCTTATATTGCTGCTGCTATGGGTA TCTGGTACCTGTGGGGACATTGTGATGTCACAGTCTCCATCCTCC CTGGCTGTGTCAACAGGAGAGAAGGTCACTATGAGCTGCAAATCC AGT**CAGAGTCTGCTCAACAGTAGAACCCGAAAGAACTAC**TT GGCTTGGTACCAGCAGAAACCAGGGCAGTCTCCTAAACTGCTGAT CTAC**TGGGCATCC**ACTAGGGAATCTGGGGTCCCTGATCGCTTCA CAGGCAGTGGATCTGGGACAGATTTCACTCTCACCATCAGCAGTG TGCAGGCTGAAGACCTGGCAGTTTATTACTGC**AAGCAATCTTAT AATCTGTACACG**TTCGGAGGGGGGACCAAGCTGGAAATAAAA | MDSQAQVLILLLLWVS GTCGDIVMSQSPSSLA VSTGEKVTMSCKSS**QS LLNSRTRKNY**LAWYQ QKPGQSPKLLIY**WAST** RESGVPDRFTGSGSGT DFTLTISSVQAEDLAV YYC**KQSYNLYT**FGGG TKLEIK |
| 301-03 IgG3 SEQ ID NO: 84, 85 | ATGAACTTCGGGCTCAGCTTGATTTTCCTTGTCCTTGTTTTTAAAA GGTGTCCAGTGTGAAGTGCAGCTGGTGGAGTCTGGGGGAGGCTTA GTGAAGCCTGGAGGGTCCCTGAAACTCTCCTGTGCAGCCTCT**GGA TTCACTTTCAGTGACTATTAC**ATGTATTGGGTTCGCCAGACTC CGGAAAAGAGGCTGGAGTGGGTCGCAACC**ATTAGTGATGGTGG TAGTTACACC**TCCTATCCAGACAGTGTGAAGGGGCGATTCACCA TCTCCAGAGACAGTGCCAAGAACAACCTGTACCTGCAAATGAGCA GTCTGAAGTCTGAGGACACAGCCATGTATTACTGT**GCAAGAGAT TACTACGGTAGTAATAGTTACACCTCGGGCTTTGCTTAC**TG GGGCCAAGGGACTCTGGTCACTGTCTCTGCA | MNFGLSLIFLVLVLKG VQCEVQLVESGGGLVK PGGSLKLSCAAS**GFTF SDYY**MYWVRQTPEKRL EWVAT**ISDGGSYT**SY PDSVKGRFTISRDSAK NNLYLQMSSLKSEDTA MYYC**ARDYYGSNSYT SGFAY**WGQGTLVTVSA |

(continued)

| Consensus DNA sequence and translated protein sequences of the variable region. The complementarity determining regions (CDRs) are underlined according to IMTG/LIGM-DB. | | |
| --- | --- | --- |
| Antibody and Isotype | Consensus cDNA Sequence | Polypeptide sequence |
| 301-03 Kappa 1 SEQ ID NO: 86, 87 | ATGGATTCACAGGCCCAGGTTCTTATATTGCTGCTGCTATGGGTA TCTGGTACCTGTGGGGACATTGTGATGTCACAGTCTCCATCCTCC CTGGCTGTGTCAGCAGGAGAGAAGGTCACTATGAGCTGCAAATCC AGT**CAGAGTCTGCTCAATAGTAGAACCCGAAAGAACTAC**TT GGCTTGGTACCAGCAGAAACCAGGGCAGTCTCCTAAACTGCTGAT CTAC**TGGGCATCC**ACTAGGGAATCTGGGGTCCCTGATCGCTTCA CAGGCAGTGGATCTGGGACAGATTTCACTCTCACCATCAGCAGTG TGCAGGCTGAAGACCTGGCAGTTTATTACTGC**AAGCAATCTTAT AATCTGTACACG**TTCGGAGGGGGGACCAAGCTGGAAATAAAA | MDSQAQVLILLLLWVS GTCGDIVMSQSPSSLA VSAGEKVTMSCKSS**QS LLNSRTRKNY**LAWYQ QKPGQSPKLLIY**WAS**T RESGVPDRFTGSGSGT DFTLTISSVQAEDLAV YYC**KQSYNLYT**FGGG TKLEIK |
| 301-03 Kappa 2 SEQ ID NO: 88, 89 | ATGAAGTTGCCTGTTAGGCTGTTGGTGCTGATGTTCTGGATTCCT GCTTCCAGCAGTGATGTTTTGATGACCCAAACTCCACTCTCCCTG CCTGTCAGTCTTGGAGATCAAGCCTCCATCTCTTGCAGATCTAGT **CAGAGCATTGTACATAGTAATGGAAACACCTAT**TTAGAATGGTAC CTGCAGAAACCAGGCCAGTCTCCAAAGCTCCTGATCTAC**AAAGTT TCC**AACCGATTTTCTGGGGTCCCAGACAGGTTCAGTGGCAGTGGA TCAGGGACAGATTTCACACTCAAGATCAGCAGAGTGGAGGCTGAG GATCTGGGAGTTTATTTCTGC**TTTCAAGGTTCACATGTTCCTCTC ACG**TTCGGTGCTGGGACCAAGCTGGAGCTGAAA | MKLPVRLLVLMFWIPA SSSDVLMTQTPLSLPV SLGDQASISCRSS**QSI VHSNGNTY**LEWYLQKP GQSPKLLIY**KVS**NRFS GVPDRFSGSGSGTDFT LKISRVEAEDLGVYFC **FQGSHVPLT**FGAGTKL ELK |
| 301-17 IgG3 SEQ ID NO: 96, 97 | ATGGGATGGAGCTGTATCATCCTCTTTTTGGTAGCAACAGCTACA GGTGTCCACTCCCAGGTCCAACTGCAGCAGCCTGGGGCTGAGCTT GTGAAGCCTGGGGCTTCAGTGAAAATGTCCTGCAAGGCTTCT**GGC TACAGCTTCACCAGCTACTGG**ATAAACTGGGTGAAGCAGAGGCCT GGACAAGGCCTTGAGTGGATTGGAGAT**GTTCATCCTGGTAGAGGT GTTTCTACC**TACAATGCGAAGTTCAAGAGCAAGGCCACACTGACT CTAGACACGTCCTCCAGCACAGCCTACATGCAGCTCAGCAGCCTG ACATCTGAGGACTCTGCGGTCTATTATTGT**TCAAGATCCCACGGT AATACCTACTGGTTCTTCGATGTC**TGGGGCGCAGGGACCACGGTC ACCGTCTCCTCAGCTACAACAACAGCCCCATCT | MGWSCIILFLVATATG VHSQVQLQQPGAELVK PGASVKMSCKAS**GYSF TSYW**INWVKQRPGQGL EWIGD**VHPGRGVST**YN AKFKSKATLTLDTSSS TAYMQLSSLTSEDSAV YYC**SRSHGNTYWFFDV** WGAGTTVTVSSATTTA PS |

EP 3 574 020 B1

22

| Consensus DNA sequence and translated protein sequences of the variable region. The complementarity determining regions (CDRs) are underlined according to IMTG/LIGM-DB. | | |
| --- | --- | --- |
| Antibody and Isotype | Consensus cDNA Sequence | Polypeptide sequence |
| 301-17 Kappa SEQ ID NO: 98, 99 | ATGAAGTTGCCTGTTAGGCTGTTGGTGCTGATGTTCTGGATTCCT GCTTCCAGCAGTGATGTTTTGATGACCCAAACTCCACTCTCCCTG CCTGTCAGTCTTGGAGATCAAGCCTCCATCTCTTGCAGATCTAGT **CAGAGCATTGTACATAGTAATGGAAACACCTAT**TTAGAATGGTAC CTGCAGAAACCAGGCCAGTCTCCAAAGCTCCTGATCTACAA**AGTT** **TCC**AACCGATTTTCTGGGGTCCCAGACAGGTTCAGTGGCAGTGGA TCAGGGACAGATTTCACACTCAAGATCAGCAGAGTGGAGGCTGAG GATCTGGGAGTTTATTACTGC**TTTCAAGGTTCACATGTTCCATTC** **ACG**TTCGGCTCGGGGACAAAGTTGGAAATAAAACGGGCTGATGCT | MKLPVRLLVLMFWIPA SSSDVLMTQTPLSLPV SLGDQASISCRSS**QSI** **VHSNGNTY**LEWYLQKP GQSPKLLIY**KVS**NRFS GVPDRFSGSGSGTDFT LKISRVEAEDLGVYYC **FQGSHVPFT**FGSGTKL EIKRADA |

EP 3 574 020 B1

**Table 4A**

| Chimeric/Humanized Antibody 301-11 |
| --- |
| VH0* SEQ ID NO: 13, 14 |
| VH1 SEQ ID NO: 15, 16 |
| VH2 SEQ ID NO: 17, 18 |
| VH3 SEQ ID NO: 19, 20 |
| VH4 SEQ ID NO: 21, 22 |
| VH5 SEQ ID NO: 23, 24 |
| VH6 SEQ ID NO: 25, 26 |
| VK0* SEQ ID NO: 27, 28 |
| VK1 SEQ ID NO: 29, 30 |
| VK2 SEQ ID NO: 31, 32 |
| VK3 SEQ ID NO: 33, 34 |
| VK4 SEQ ID NO: 35, 36 |
| VK5 SEQ ID NO: 37, 38 |
| VK6 SEQ ID NO: 39, 40 |

**Table 4B**

| Humanized = Antibody | cDNA Sequence | Polypeptide Sequence |
|---|---|---|
| 301-17<br>VH0*<br>SEQ ID NO: 41,42 | CAGGTCCAACTGCAGCAGCCTGGGGCTGAGCTTGTGAAGCCTGGG<br>GCTTCAGTGAAGATGTCCTGCAAGGCTTCT**GGCTACAGCTTCACC**<br>**AGCTACTGG**ATAAACTGGGTGAAGCAGAGGCCTGGACAAGGCCTT<br>GAGTGGATTGGAGAT**GTGCATCCTGGTAGAGGCGTGTCCACA**TAC<br>AATGCTAAGTTCAAGAGCAAGGCCACACTGACTCTGGACACATCC<br>TCCAGCACAGCCTACATGCAGCTCAGCAGCCTGACATCTGAGGAC<br>TCTGCGGTCTATTACTGT**AGCAGATCCCATGGTAACACCTACTGG**<br>**TTTTTTGACGTC**TGGGGCGCAGGCACCACGGTCACCGTCTCCTCA | QVQLQQPGAELVKPGA<br>SVKMSCKAS**GYSFTSY**<br>**W**INWVKQRPGQGLEWI<br>GD**VHPGRGVST**YNAKF<br>KSKATLTLDTSSSTAY<br>MQLSSLTSEDSAVYYC<br>**SRSHGNTYWFFDV**WGA<br>GTTVTVSS |
| VH1<br>SEQ ID NO: 43, 44 | CAGGTCCAACTGGTGCAGTCTGGGGCTGAGCTTAAGAAGCCTGGG<br>GCTTCAGTGAAGATGTCCTGCAAGGCTTCT**GGCTACAGCTTCACC**<br>**AGCTACTGG**ATAAACTGGGTGAAGCAGAGGCCTGGACAAGGCCTT<br>GAGTGGATTGGAGAT**GTGCATCCTGGTAGAGGCGTGTCCACA**TAC<br>AATGCTAAGTTCAAGAGCAGAGCCACACTGACTCTGGACACATCC<br>ATAAGCACAGCCTACATGCAGCTCAGCAGCCTGACATCTGAGGAC<br>TCTGCGGTCTATTACTGT**AGCAGATCCCATGGTAACACCTACTGG**<br>**TTTTTTGACGTC**TGGGGCCAAGGCACCACGGTCACCGTCTCCTCA | QVQLVQSGAELKKPGA<br>SVKMSCKAS**GYSFTSY**<br>**W**INWVKQRPGQGLEWI<br>GD**VHPGRGVST**YNAKF<br>KSRATLTLDTSISTAY<br>MQLSSLTSEDSAVYYC<br>**SRSHGNTYWFFDV**WGQ<br>GTTVTVSS |
| VH2<br>SEQ ID NO: 45, 46 | CAGGTCCAACTGGTGCAGTCTGGGGCTGAGGTGAAGAAGCCTGGG<br>GCTTCAGTGAAGATGTCCTGCAAGGCTTCT**GGCTACAGCTTCACC**<br>**AGCTACTGG**ATAAACTGGGTGAAGCAGAGGCCTGGACAAGGCCTT<br>GAGTGGATTGGAGAT**GTGCATCCTGGTAGAGGCGTGTCCACA**TAC<br>AATGCTAAGTTCAAGAGCAGAGCCACACTGACTCTGGACACATCC<br>ATAAGCACAGCCTACATGGAGCTCAGCAGCCTGAGATCTGAGGAC<br>ACGGCGGTCTATTACTGT**AGCAGATCCCATGGTAACACCTACTGG**<br>**TTTTTTGACGTC**TGGGGCCAAGGCACCACGGTCACCGTCTCCTCA | QVQLVQSGAEVKKPGA<br>SVKMSCKAS**GYSFTSY**<br>**W**INWVKQRPGQGLEWI<br>GD**VHPGRGVST**YNAKF<br>KSRATLTLDTSISTAY<br>MELSSLRSEDTAVYYC<br>**SRSHGNTYWFFDV**WGQ<br>GTTVTVSS |
| VH3<br>SEQ ID NO: 47, 48 | CAGGTCCAACTGGTGCAGTCTGGGGCTGAGGTGAAGAAGCCTGGG<br>GCTTCAGTGAAGGTGTCCTGCAAGGCTTCT**GGCTACAGCTTCACC**<br>**AGCTACTGG**ATAAACTGGGTGCGACAGAGGCCTGGACAAGGCCTT<br>GAGTGGATTGGAGAT**GTGCATCCTGGTAGAGGCGTGTCCACA**TAC<br>AATGCTAAGTTCAAGAGCAGAGCCACACTGACTCTGGACACATCC<br>ATAAGCACAGCCTACATGGAGCTCAGCAGCCTGAGATCTGAGGAC<br>ACGGCGGTCTATTACTGT**AGCAGATCCCATGGTAACACCTACTGG**<br>**TTTTTTGACGTC**TGGGGCCAAGGCACCACGGTCACCGTCTCCTCA | QVQLVQSGAEVKKPGA<br>SVKVSCKAS**GYSFTSY**<br>**W**INWVRQRPGQGLEWI<br>GD**VHPGRGVST**YNAKF<br>KSRATLTLDTSISTAY<br>MELSSLRSEDTAVYYC<br>**SRSHGNTYWFFDV**WGQ<br>GTTVTVSS |

(continued)

| Humanized = Antibody | cDNA Sequence | Polypeptide Sequence |
|---|---|---|
| VH4<br>SEQ ID NO: 49, 50 | CAGGTCCAACTGGTGCAGTCTGGGGCTGAGGTGAAGAAGCCTGGG<br>GCTTCAGTGAAGGTGTCCTGCAAGGCTTCT**GGCTACAGCTTCACC**<br>**AGCTACTGG**ATAAACTGGGTGCGACAGAGGCCTGGACAAGGCCTT<br>GAGTGGATTGGAGAT**GTGCATCCTGGTAGAGGCGTGTCCACA**TAC<br>AATGCTAAGTTCAAGAGCAGAGTCACACTGACTCTGGACACATCC<br>ATAAGCACAGCCTACATGGAGCTCAGCAGCCTGAGATCTGAGGAC<br>ACGGCGGTCTATTACTGT**AGCAGATCCCATGGTAACACCTACTGG**<br>**TTTTTTGACGTC**TGGGGCCAAGGCACCACGGTCACCGTCTCCTCA | QVQLVQSGAEVKKPGA<br>SVKVSCKAS**GYSFTSY**<br>**W**INWVRQRPGQGLEWI<br>GD**VHPGRGVST**YNAKF<br>KSRVTLTLDTSISTAY<br>MELSSLRSEDTAVYYC<br>**SRSHGNTYWFFDV**WGQ<br>GTTVTVSS |
| VH5<br>SEQ ID NO: 51, 52 | CAGGTCCAACTGGTGCAGTCTGGGGCTGAGGTGAAGAAGCCTGGG<br>GCTTCAGTGAAGGTGTCCTGCAAGGCTTCT**GGCTACAGCTTCACC**<br>**AGCTACTGG**ATAAACTGGGTGCGACAGAGGCCTGGACAAGGCCTT<br>GAGTGGATGGGAGAT**GTGCATCCTGGTAGAGGCGTGTCCACA**TAC<br>AATGCTAAGTTCAAGAGCAGAGTCACACTGACTAGGGACACATCC<br>ATAAGCACAGCCTACATGGAGCTCAGCAGCCTGAGATCTGAGGAC<br>ACGGCGGTCTATTACTGT**AGCAGATCCCATGGTAACACCTACTGG**<br>**TTTTTTGACGTC**TGGGGCCAAGGCACCACGGTCACCGTCTCCTCA | QVQLVQSGAEVKKPGA<br>SVKVSCKAS**GYSFTSY**<br>**W**INWVRQRPGQGLEWM<br>GD**VHPGRGVST**YNAKF<br>KSRVTLTRDTSISTAY<br>MELSSLRSEDTAVYYC<br>**SRSHGNTYWFFDV**WGQ<br>GTTVTVSS |
| VH6<br>SEQ ID NO: | CAGGTCCAACTGGTGCAGTCTGGGGCTGAGGTGAAGAAGCCTGGG<br>GCTTCAGTGAAGGTGTCCTGCAAGGCTTCT**GGCTACAGCTTCACC**<br>**AGCTACTGG**ATAAACTGGGTGCGACAGAGGCCTGGACAAGGCCTT | QVQLVQSGAEVKKPGA<br>SVKVSCKAS**GYSFTSY**<br>**W**INWVRQRPGQGLEWM |
| 53, 54 | GAGTGGATGGGAGAT**GTGCATCCTGGTAGAGGCGTGTCCACA**TAC<br>AATGCTAAGTTCCAGGGCAGAGTCACAATGACTAGGGACACATCC<br>ATAAGCACAGCCTACATGGAGCTCAGCAGCCTGAGATCTGAGGAC<br>ACGGCGGTCTATTACTGT**AGCAGATCCCATGGTAACACCTACTGG**<br>**TTTTTTGACGTC**TGGGGCCAAGGCACCACGGTCACCGTCTCCTCA | GD**VHPGRGVST**YNAKF<br>QGRVTMTRDTSISTAY<br>MELSSLRSEDTAVYYC<br>**SRSHGNTYWFFDV**WGQ<br>GTTVTVSS |
| VK0*<br>SEQ ID NO:<br>55, 56 | GATGTTTTGATGACCCAAACTCCACTCTCCCTGCCTGTCAGTCTT<br>GGAGATCAAGCCTCCATCTCTTGCAGATCTAGT**CAGAGCATTGTA**<br>**CATAGTAATGGAAACACCTAT**TTAGAATGGTACCTGCAGAAACCA<br>GGCCAGTCTCCAAAGCTCCTGATCTAC**AAAGTTTCC**AACCGATTT<br>TCTGGGGTCCCAGACAGGTTCAGTGGCAGTGGATCAGGGACAGAT<br>TTCACACTCAAGATCAGCAGAGTGGAGGCTGAGGATCTGGGAGTT<br>TATTACTGC**TTTCAAGGTTCACATGTTCCTTTCACT**TTTGGCAGC<br>GGGACCAAGCTGGAGATCAAA | DVLMTQTPLSLPVSLG<br>DQASISCRSS**QSIVHS**<br>**NGNTY**LEWYLQKPGQS<br>PKLLIY**KVS**NRFSGVP<br>DRFSGSGSGTDFTLKI<br>SRVEAEDLGVYYC**FQG**<br>**SHVPFT**FGSGTKLEIK |

| Humanized = Antibody | cDNA Sequence | Polypeptide Sequence |
|---|---|---|
| VK1 SEQ ID NO: 57, 58 | GATGTTTTGATGACCCAATCTCCACTCTCCCTGCCTGTCACCCTT GGACAGCCGGCCTCCATCTCTTGCAGATCTAGT**CAGAGCATTGTA CATAGTAATGGAAACACCTAT**TTAGAATGGTTTCAGCAGAAACCA GGCCAGTCTCCAAGGCGCCTGATCTAC**AAAGTTTCC**AACCGATTT TCTGGGGTCCCAGACAGGTTCAGTGGCAGTGGATCAGGGACAGAT TTCACACTCAAGATCAGCAGAGTGGAGGCTGAGGATGTTGGAGTT TATTACTGC**TTTCAAGGTTCACATGTTCCTTTCACT**TTTGGCCAA GGGACCAAGCTGGAGATCAAA | DVLMTQSPLSLPVTLG QPASISCRSS**QSIVHS NGNTY**LEWFQQKPGQS PRRLIY**KVS**NRFSGVP DRFSGSGSGTDFTLKI SRVEAEDVGVYYC**FQG SHVPFT**FGQGTKLEIK |
| VK2 SEQ ID NO: 59, 60 | GATGTTGTGATGACCCAATCTCCACTCTCCCTGCCTGTCACCCTT GGACAGCCGGCCTCCATCTCTTGCAGATCTAGT**CAGAGCATTGTA CATAGTAATGGAAACACCTAT**TTAGAATGGTTTCAGCAGAAACCA GGCCAGTCTCCAAGGCGCCTGATCTAC**AAAGTTTCC**AACCGATTT TCTGGGGTCCCAGACAGGTTCAGTGGCAGTGGATCAGGGACAGAT TTCACACTCAAGATCAGCAGAGTGGAGGCTGAGGATGTTGGAGTT TATTACTGC**TTTCAAGGTTCACATGTTCCTTTCACT**TTTGGCCAA GGGACCAAGCTGGAGATCAAA | DVVMTQSPLSLPVTLG QPASISCRSS**QSIVHS NGNTY**LEWFQQKPGQS PRRLIY**KVS**NRFSGVP DRFSGSGSGTDFTLKI SRVEAEDVGVYYC**FQG SHVPFT**FGQGTKLEIK |
| VK3 SEQ ID NO: 61,62 | GATGTTGTGATGACCCAATCTCCACTCTCCCTGCCTGTCACCCTT GGACAGCCGGCCTCCATCTCTTGCAGATCTAGT**CAGAGCATTGTA CATAGTAATGGAAACACCTAT**TTAGAATGGTTTCAGCAGAGGCCA GGCCAGTCTCCAAGGCGCCTGATCTAC**AAAGTTTCC**AACCGATTT TCTGGGGTCCCAGACAGGTTCAGTGGCAGTGGATCAGGGACAGAT TTCACACTCAAGATCAGCAGAGTGGAGGCTGAGGATGTTGGAGTT TATTACTGC**TTTCAAGGTTCACATGTTCCTTTCACT**TTTGGCCAA GGGACCAAGCTGGAGATCAAA | DVVMTQSPLSLPVTLG QPASISCRSS**QSIVHS NGNTY**LEWFQQRPGQS PRRLIY**KVS**NRFSGVP DRFSGSGSGTDFTLKI SRVEAEDVGVYYC**FQG SHVPFT**FGQGTKLEIK |
| VK4 SEQ ID NO: 63, 64 | GATGTTCTGATGACCCAATCTCCACTCTCCCTGCCTGTCACCCTT GGACAGCCGGCCTCCATCTCTTGCAGATCTAGT**CAGAGCATTGTA CATAGTAATGGAAACACCTAT**TTAGAATGGTACCTGCAGAGGCCA GGCCAGTCTCCAAAGCTGCTGATCTAC**AAAGTTTCC**AACCGATTT TCTGGGGTCCCAGACAGGTTCAGTGGCAGTGGATCAGGGACAGAT TTCACACTCAAGATCAGCAGAGTGGAGGCTGAGGATGTTGGAGTT TATTACTGC**TTTCAAGGTTCACATGTTCCTTTCACT**TTTGGCCAA GGGACCAAGCTGGAGATCAAA | DVLMTQSPLSLPVTLG QPASISCRSS**QSIVHS NGNTY**LEWYLQRPGQS PKLLIY**KVS**NRFSGVP DRFSGSGSGTDFTLKI SRVEAEDVGVYYC**FQG SHVPFT**FGQGTKLEIK |

(continued)

| Humanized = Antibody | cDNA Sequence | Polypeptide Sequence |
|---|---|---|
| VK5<br>SEQ ID NO:<br>65, 66 | GATGTTCTGATGACCCAATCTCCACTCTCCCTGCCTGTCACCCTT<br>GGACAGCCGGCCTCCATCTCTTGCAGATCTAGT**CAGAGCATTGTA**<br>**CATAGTAATGGAAACACCTAT**TTAGAATGGTACCAGCAGAGGCCA<br>GGCCAGTCTCCAAGGCTGCTGATCTAC**AAAGTTTCC**AACCGATTT<br>TCTGGGGTCCCAGACAGGTTCAGTGGCAGTGGATCAGGGACAGAT<br>TTCACACTCAAGATCAGCAGAGTGGAGGCTGAGGATGTTGGAGTT<br>TATTACTGC**TTTCAAGGTTCACATGTTCCTTTCACT**TTTGGCCAA<br>GGGACCAAGCTGGAGATCAAA | DVLMTQSPLSLPVTLG<br>QPASISCRSS**QSIVHS**<br>**NGNTY**LEWYQQRPGQS<br>PRLLIY**KVS**NRFSGVP<br>DRFSGSGSGTDFTLKI<br>SRVEAEDVGVYYC**FQG**<br>**SHVPFT**FGQGTKLEIK |
| VK6<br>SEQ ID NO:<br>67, 68 | GATGTTGTGATGACCCAATCTCCACTCTCCCTGCCTGTCACCCTT<br>GGACAGCCGGCCTCCATCTCTTGCAGATCTAGT**CAGAGCATTGTA**<br>**CATAGTAATGGAAACACCTAT**TTAGAATGGTACCAGCAGAGGCCA<br>GGCCAGTCTCCAAGGCTGCTGATCTAC**AAAGTTTCC**AACCGATTT<br>TCTGGGGTCCCAGACAGGTTCAGTGGCAGTGGATCAGGGACAGAT<br>TTCACACTCAAGATCAGCAGAGTGGAGGCTGAGGATGTTGGAGTT | DVVMTQSPLSLPVTLG<br>QPASISCRSS**QSIVHS**<br>**NGNTY**LEWYQQRPGQS<br>PRLLIY**KVS**NRFSGVP<br>DRFSGSGSGTDFTLKI |
| | TATTACTGC**TTTCAAGGTTCACATGTTCCTTTCACT**TTTGGCCAA<br>GGGACCAAGCTGGAGATCAAA | SRVEAEDVGVYYC**FQG**<br>**SHVPFT**FGQGTKLEIK |
| ***VH0 and VK0** denotes chimeric antibodies comprised of the human constant domain and mouse variable domain sequences | | |

**Table 5 Humanized antibody IgG4 sequence**

| Constant regions | cDNA Sequence | Polypeptide sequence |
|---|---|---|
| IgG4 heavy chain SEQ ID NO: 69, 70 | GCTTCCACCAAGGGCCCATCCGTCTTCCCCCTGGCGCCCTGCTCC AGGAGCACCTCCGAGAGCACAGCCGCCCTGGGCTGCCTGGTCAAG GACTACTTCCCCGAACCGGTGACGGTGTCGTGGAACTCAGGCGCC CTGACCAGCGGCGTGCACACCTTCCCGGCTGTCCTACAGTCCTCA GGACTCTACTCCCTCAGCAGCGTGGTGACCGTGCCCTCCAGCAGC TTGGGCACGAAGACCTACACCTGCAATGTAGATCACAAGCCCAGC AACACCAAGGTGGACAAGAGAGTTGAGTCCAAATATGGTCCCCCA TGCCCACCATGCCCAGCACCTGAGTTCCTGGGGGGACCATCAGTC TTCCTGTTCCCCCCAAAACCCAAGGACACTCTCATGATCTCCCGG ACCCCTGAGGTCACGTGCGTGGTGGTGGACGTGAGCCAGGAAGAC CCCGAGGTCCAGTTCAACTGGTACGTGGATGGCGTGGAGGTGCAT AATGCCAAGACAAAGCCGCGGGAGGAGCAGTTCAACAGCACGTAC CGTGTGGTCAGCGTCCTCACCGTCCTGCACCAGGACTGGCTGAAC GGCAAGGAGTACAAGTGCAAGGTCTCCAACAAAGGCCTCCCGTCC TCCATCGAGAAAACCATCTCCAAAGCCAAAGGGCAGCCCCGAGAG CCACAGGTGTACACCCTGCCCCCATCCCAGGAGGAGATGACCAAG AACCAGGTCAGCCTGACCTGCCTGGTCAAAGGCTTCTACCCCAGC GACATCGCCGTGGAGTGGGAGAGCAATGGGCAGCCGGAGAACAAC TACAAGACCACGCCTCCCGTGCTGGACTCCGACGGCTCCTTCTTC CTCTACAGCAGGCTAACCGTGGACAAGAGCAGGTGGCAGGAGGGG AATGTCTTCTCATGCTCCGTGATGCATGAGGCTCTGCACAACCAC TACACACAGAAGAGCCTCTCCCTGTCTCTGGGTAAATGA | ASTKGPSVFPLAPCSR STSESTAALGCLVKDY FPEPVTVSWNSGALTS GVHTFPAVLQSSGLYS LSSVVTVPSSSLGTKT YTCNVDHKPSNTKVDK RVESKYGPPCPPCPAP EFLGGPSVFLFPPKPK DTLMISRTPEVTCVVV DVSQEDPEVQFNWYVD GVEVHNAKTKPREEQF NSTYRVVSVLTVLHQD WLNGKEYKCKVSNKGL PSSIEKTISKAKGQPR EPQVYTLPPSQEEMTK NQVSLTCLVKGFYPSD IAVEWESNGQPENNYK TTPPVLDSDGSFFLYS RLTVDKSRWQEGNVFS CSVMHEALHNHYTQKS LSLSLGK |
| **Kappa** SEQ ID NO: 71, 72 | CGAACTGTGGCTGCACCATCTGTCTTCATCTTCCCGCCATCTGAT GAGCAGTTGAAATCTGGAACTGCCTCTGTTGTGTGCCTGCTGAAT AACTTCTATCCCAGAGAGGCCAAAGTACAGTGGAAGGTGGATAAC GCCCTCCAATCGGGTAACTCCCAGGAGAGTGTCACAGAGCAGGAC AGCAAGGACAGCACCTACAGCCTCAGCAGCACCCTGACGCTGAGC AAAGCAGACTACGAGAAACACAAAGTCTACGCCTGCGAAGTCACC CATCAGGGCCTGAGCTCGCCCGTCACAAAGAGCTTCAACAGGGGA GAGTGTTAG | RTVAAPSVFIFPPSDE QLKSGTASVVCLLNNF YPREAKVQWKVDNALQ SGNSQESVTEQDSKDS TYSLSSTLTLSKADYE KHKVYACEVTHQGLSS PVTKSFNRGEC |

## Example 2

### Immunohistochemistry

Immunohistochemistry was performed on frozen human brain sections, with no fixation or antigen retrieval. In a humidified chamber, non-specific staining was blocked by incubation with serum-free protein blocking reagent (Dako Canada Inc., Mississauga, ON, Canada) for 1 h. The following primary antibodies were used for immunostaining: mouse monoclonal isotype controls IgG1, IgG2a, and IgG2b, and anti-amyloidβ 6E10, all purchased from Biolegend, and purified antibodies 301-11 and 301-17. All antibodies were used at 1 μg/mL. Sections were incubated at room temperature for 1h, and washed 3 × 5 min in TBS-T. Anti-Mouse IgG conjugated to Horseradish Peroxidase (1:1000) was applied to sections and incubated 45 min, then washed 3 × 5 min in TBS-T. DAB chromogen reagent (Vector Laboratories, Burlington ON, Canada) was applied and sections rinsed with distilled water when the desired level of target to background staining was achieved. Sections were counterstained with Mayer's haematoxylin, dehydrated and cover slips were applied. Slides were examined under a light microscope (Zeiss Axiovert 200M, Carl Zeiss Canada, Toronto ON, Canada) and representative images captured at 20 and 40X magnification using a Leica DC300 digital camera and software (Leica Microsystems Canada Inc., Richmond Hill, ON). Images were optimized in Adobe Photoshop using Levels Auto Correction.

### Brain Extracts

Human brain tissues were obtained from the University of Maryland Brain and Tissue Bank upon approval from the UBC Clinical Research Ethics Board (C04-0595). Clinical diagnosis of probable AD was based on NINCDS-ADRDA criteria [5].

*Homogenization:* Human brain tissue samples were weighed and subsequently submersed in a volume of fresh, ice cold TBS and EDTA-free protease inhibitor cocktail from Roche Diagnostics (Laval QC, Canada) such that the final concentration of brain tissue was 20% (w/v). Tissue was homogenized in this buffer using a mechanical probe homogenizer (3 × 30 sec pulses with 30 sec pauses in between, all performed on ice). TBS homogenized samples were then subjected to ultracentrifugation (70,000xg for 90 min). Supernatants were collected, aliquoted and stored at -80°C. The protein concentration of TBS homogenates was determined using a BCA protein assay (Pierce Biotechnology Inc, Rockford IL, USA).

Positive binding in brain extracts was confirmed using antibody 6E10.

*SPR Analysis:* 4 brain extracts from AD patients and 4 brain extracts from age-matched controls were pooled and analyzed. Brain samples, homogenized in TBS, included frontal cortex Brodmann area 9. All experiments were performed using a Molecular Affinity Screening System (MASS-1) (Sierra Sensors GmbH, Hamburg, Germany), an analytical biosensor that employs high intensity laser light and high speed optical scanning to monitor binding interactions in real time. Purified antibodies generated for cyclopeptides described herein were captured on separate flow cells of a protein A-derivatized sensor chip and diluted samples injected over the surfaces for 180 seconds, followed by 120 seconds of dissociation in buffer and surface regeneration. Binding responses were double-referenced by subtraction of mouse control IgG reference surface binding and assay buffer, and the different groups of samples compared.

## Results

### Brain Extracts, CSF and Immunohistochemistry

The antibodies were tested for their ability to bind A-beta in soluble brain extracts, CSF and tissue samples of cavaderic healthy control and AD brains, results are shown in Table 6. Strength of positivity in Table 6 is shown by the number plus signs.

Each of antibodies 301-11 and 301-17 showed positive binding with brain homogenates and CSF from AD patients compared to control patients.

As shown in Table 6, the purified antibodies showed preferential binding to AD vs non-AD in soluble brain extracts and CSF, and did not appreciably bind to plaque fibrils by IHC.

**Table 6: Summary of binding characteristics**

| Antibody | Oligomers/ Monomers | Brain Extract AD/Non-AD | IHC - Plaque Staining (Frozen Section Brain 1630) | CSF |
|---|---|---|---|---|
| 301-3 | ++ | ++ | - | + |
| 301-11 | ++ | +++ | - | ++ |
| 301-17 | ++ | ++ | - | + |
| * Scoring is relative to other clones not shown herein in the same sample category. | | | | |

## Example 3

### Binding to A-beta synthetic oligomers.

To further verify and validate A-beta42 Oligomer binding, purified antibodies were covalently immobilized to a sensorchip, followed by the injection over the surface of commercially-prepared stable A-beta42 Oligomers (1microM) (SynAging SAS, Vandœuvre-lès-Nancy, France).

Antibodies 301-3, 301-11 and 307-17, bound the stable A-beta 42 oligomers (1 microM) with binding response units (BRUs) of an average of 14.5 (301-3), 19.3 (301-11) and 30 (301-17), respectively. By comparison, the negative control IgG1 did not meaningfully bind to the oligomers (mean binding of BRU 2.5) while the pan-A$\beta$ positive control antibody 6E10 bound with an average BRU of 90.

## Example 4

### Immunohistochemistry on Formalin Fixed Tissues

Human AD brain tissue sections were assessed using antibodies 301-11, 301-17. The patient had been previously characterized and diagnosed with Alzheimer's disease with a tripartite approach: (i) Bielschowsky silver method to demonstrate senile plaques and neurofibrillary tangles, (ii) Congo red to demonstrate amyloid and (iii) tau immunohistochemistry to demonstrate tangles and to confirm the senile plaques are "neuritic". This tissue was used to test plaque reactivity of selected monoclonal antibody clones. The brain tissues were fixed in 10% buffered formalin for several days and paraffin processed in the Sakura VIP tissue processors. Tissue sections were probed with $1\mu$g/ml of antibody with and without microwave antigen retrieval (AR). The pan-amyloid beta reactive antibody 6E10 was included along with selected antibody clones as a positive control. Antibodies were diluted in Antibody Diluent (Ventana), color was developed with OptiView DAB (Ventana). The staining was performed on the Ventana Benchmark XT IHC stainer. Images were obtained with an Olympus BX45 microscope. Images were analyzed blind by a professional pathologist with expertise in neuropathology.

As shown in Table 7 below, using fixed tissue, the tested antibodies were negative for specific staining of senile plaque amyloid. The 6E10 antibody, used as the positive control, showed strong plaque staining.

**Table 7**

|  | Antibodies | Staining of senile plaque amyloid |
|---|---|---|
|  | 301-11 | Neg |
|  | 301-17 | Neg |
| Positive Control | 6E10 | strongly positive |

## Example 5

### Recombinant IgG1 and IgG2a antibodies

Recombinant IgG1 and IgG2a 301-17 construct were made by grafting the CDRs of hybridoma-derived 301-17 onto a murine IgG1 or IgG2a backbone (WuXi, Biologics). The sequences are provided in Table 8.

**Table 8** - **Heavy chain and light chain Sequences for 301-17 Isotypes**

| Antibody and Isotype | cDNA Sequence | Polypeptide sequence |
|---|---|---|
| 301-17 IgG1 SEQ ID NO: 90, 91 | CAGGTGCAGCTGCAGCAGCCTGGCGCTGAGCTGGTGAAGCCTGGA GCCTCCGTGAAGATGTCCTGCAAGGCCTCCGGCTACTCCTTCACC AGCTACTGGATCAACTGGGTGAAGCAGAGGCCCGGACAGGGCCTG GAGTGGATTGGAGACGTGCACCCTGGCCGGGGAGTGTCCACCTAC AACGCCAAGTTCAAGTCCAAGGCCACCCTGACCCTGGACACCTCC AGCTCCACCGCCTACATGCAGCTGTCCTCCCTGACCTCCGAGGAC TCCGCCGTGTACTACTGCAGCAGGTCCCACGGCAACACCTACTGG TTTTTCGACGTGTGGGGCGCCGGAACCACAGTGACCGTGTCCTCC GCCAAAACGACACCCCCATCTGTCTATCCACTGGCCCCTGGATCT GCTGCCCAAACTAACTCCATGGTGACCCTGGGATGCCTGGTCAAG GGCTATTTCCCTGAGCCAGTGACAGTGACCTGGAACTCTGGATCC CTGTCCAGCGGTGTGCACACCTTCCCAGCTGTCCTGGAGTCTGAC CTCTACACTCTGAGCAGCTCAGTGACTGTCCCCTCCAGCCCTCGG CCCAGCGAGACCGTCACCTGCAACGTTGCCCACCCGGCCAGCAGC ACCAAGGTGGACAAGAAAATTGTGCCCAGGGATTGTGGTTGTAAG CCTTGCATATGTACAGTCCCAGAAGTATCATCTGTCTTCATCTTC CCCCCAAAGCCCAAGGATGTGCTCACCATTACTCTGACTCCTAAG GTCACGTGTGTTGTGGTAGACATCAGCAAGGATGATCCCGAGGTC CAGTTCAGCTGGTTTGTAGATGATGTGGAGGTGCACACAGCTCAG ACGCAACCCCGGGAGGAGCAGTTCAACAGCACTTTCCGCTCAGTC AGTGAACTTCCCATCATGCACCAGGACTGGCTCAATGGCAAGGAG TTCAAATGCAGGGTCAACAGTGCAGCTTTCCCTGCCCCCATCGAG AAAACCATCTCCAAAACCAAAGGCAGACCGAAGGCTCCACAGGTG TACACCATTCCACCTCCCAAGGAGCAGATGGCCAAGGATAAAGTC AGTCTGACCTGCATGATAACAGACTTCTTCCCTGAAGACATTACT GTGGAGTGGCAGTGGAATGGGCAGCCAGCGGAGAACTACAAGAAC ACTCAGCCCATCATGAACACGAATGGCTCTTACTTCGTCTACAGC AAGCTCAATGTGCAGAAGAGCAACTGGGAGGCAGGAAATACTTTC ACCTGCTCTGTGTTACATGAGGGCCTGCACAACCACCATACTGAG AAGAGCCTCTCCCACTCTCCTGGTAAATGATGA | QVQLQQPGAELVKPGA SVKMSCKASGYSFTSY WINWVKQRPGQGLEWI GDVHPGRGVSTYNAKF KSKATLTLDTSSSTAY MQLSSLTSEDSAVYYC SRSHGNTYWFFDVWGA GTTVTVSSAKTTPPSV YPLAPGSAAQTNSMVT LGCLVKGYFPEPVTVT WNSGSLSSGVHTFPAV LESDLYTLSSSVTVPS SPRPSETVTCNVAHPA SSTKVDKKIVPRDCGC KPCICTVPEVSSVFIF PPKPKDVLTITLTPKV TCVVVDISKDDPEVQF SWFVDDVEVHTAQTQP REEQFNSTFRSVSELP IMHQDWLNGKEFKCRV NSAAFPAPIEKTISKT KGRPKAPQVYTIPPPK EQMAKDKVSLTCMITD FFPEDITVEWQWNGQP AENYKNTQPIMNTNGS YFVYSKLNVQKSNWEA GNTFTCSVLHEGLHNH HTEKSLSHSPGK |

(continued)

| Antibody and Isotype | cDNA Sequence | Polypeptide sequence |
|---|---|---|
| 301-17 IgG2a SEQ ID NO: 92, 93 | CAGGTGCAGCTGCAGCAGCCTGGCGCTGAGCTGGTGAAGCCTGGA GCCTCCGTGAAGATGTCCTGCAAGGCCTCCGGCTACTCCTTCACC AGCTACTGGATCAACTGGGTGAAGCAGAGGCCCGGACAGGGCCTG GAGTGGATTGGAGACGTGCACCCTGGCCGGGGAGTGTCCACCTAC AACGCCAAGTTCAAGTCCAAGGCCACCCTGACCCTGGACACCTCC AGCTCCACCGCCTACATGCAGCTGTCCTCCCTGACCTCCGAGGAC TCCGCCGTGTACTACTGCAGCAGGTCCCACGGCAACACCTACTGG TTTTTCGACGTGTGGGGCGCCGGAACCACAGTGACCGTGTCCTCC GCCAAAACAACAGCCCCATCGGTCTATCCACTGGCCCCTGTGTGT GGAGATACAACTGGCTCCTCGGTGACTCTAGGATGCCTGGTCAAG GGTTATTTCCCTGAGCCAGTGACCTTGACCTGGAACTCTGGATCC CTGTCCAGTGGTGTGCACACCTTCCCAGCTGTCCTGCAGTCTGAC CTCTACACCCTCAGCAGCTCAGTGACTGTAACCTCGAGCACCTGG CCCAGCCAGTCCATCACCTGCAATGTGGCCCACCCGGCAAGCAGC ACCAAGGTGGACAAGAAAATTGAGCCCAGAGGGCCCACAATCAAG CCCTGTCCTCCATGCAAATGCCCAGCACCTAACCTCTTGGGTGGA CCATCCGTCTTCATCTTCCCTCCAAAGATCAAGGATGTACTCATG ATCTCCCTGAGCCCCATAGTCACATGTGTGGTGGTGGATGTGAGC GAGGATGACCCAGATGTCCAGATCAGCTGGTTTGTGAACAACGTG GAAGTACACACAGCTCAGACACAAACCCATAGAGAGGATTACAAC AGTACTCTCCGGGTGGTCAGTGCCCTCCCCATCCAGCACCAGGAC TGGATGAGTGGCAAGGAGTTCAAATGCAAGGTCAACAACAAAGAC CTCCCAGCGCCCATCGAGAGAACCATCTCAAAACCCAAAGGGTCA GTAAGAGCTCCACAGGTATATGTCTTGCCTCCACCAGAAGAAGAG ATGACTAAGAAACAGGTCACTCTGACCTGCATGGTCACAGACTTC ATGCCTGAAGACATTTACGTGGAGTGGACCAACAACGGGAAAACA GAGCTAAACTACAAGAACACTGAACCAGTCCTGGACTCTGATGGT TCTTACTTCATGTACAGCAAGCTGAGAGTGGAAAAGAAGAACTGG GTGGAAAGAAATAGCTACTCCTGTTCAGTGGTCCACGAGGGTCTG CACAATCACCACACGACTAAGAGCTTCTCCCGGACTCCGGGTAAA TGATGA | QVQLQQPGAELVKPGA SVKMSCKASGYSFTSY WINWVKQRPGQGLEWI GDVHPGRGVSTYNAKF KSKATLTLDTSSSTAY MQLSSLTSEDSAVYYC SRSHGNTYWFFDVWGA GTTVTVSSAKTTAPSV YPLAPVCGDTTGSSVT LGCLVKGYFPEPVTLT WNSGSLSSGVHTFPAV LQSDLYTLSSSVTVTS STWPSQSITCNVAHPA SSTKVDKKIEPRGPTI KPCPPCKCPAPNLLGG PSVFIFPPKIKDVLMI SLSPIVTCVVVDVSED DPDVQISWFVNNVEVH TAQTQTHREDYNSTLR VVSALPIQHQDWMSGK EFKCKVNNKDLPAPIE RTISKPKGSVRAPQVY VLPPPEEEMTKKQVTL TCMVTDFMPEDIYVEW TNNGKTELNYKNTEPV LDSDGSYFMYSKLRVE KKNWVERNSYSCSVVH EGLHNHHTTKSFSRTP GK |
| 301-17 Kappa SEQ ID NO: 94, 95 | GATGTGCTGATGACCCAGACCCCTCTGTCCCTGCCTGTGTCCCTG GGCGATCAGGCCAGCATCTCCTGCAGGTCCTCCCAGTCCATCGTG CACTCCAACGGCAACACCTACCTGGAGTGGTACCTGCAGAAGCCC GGCCAGTCCCCCAAGCTGCTGATCTACAAGGTGTCCAACCGGTTC TCCGGCGTGCCCGATAGGTTCTCCGGATCCGGCTCCGGCACCGAC TTTACCCTGAAGATCTCCAGGGTGGAGGCCGAGGACCTGGGCGTG TACTACTGCTTTCAGGGCTCCCACGTGCCCTTCACCTTCGGCTCC GGCACCAAGCTGGAGATCAAGCGGGCTGATGCTGCACCAACTGTA TCCATCTTCCCACCATCCAGTGAGCAGTTAACATCTGGAGGTGCC TCAGTCGTGTGCTTCTTGAACAACTTCTACCCCAAAGACATCAAT GTCAAGTGGAAGATTGATGGCAGTGAACGACAAAATGGCGTCCTG AACAGTTGGACTGATCAGGACAGCAAAGACAGCACCTACAGCATG AGCAGCACCCTCACGTTGACCAAGGACGAGTATGAACGACATAAC AGCTATACCTGTGAGGCCACTCACAAGACATCAACTTCACCCATT GTCAAGAGCTTCAACAGGAATGAGTGTTGATGA | DVLMTQTPLSLPVSLG DQASISCRSSQSIVHS NGNTYLEWYLQKPGQS PKLLIYKVSNRFSGVP DRFSGSGSGTDFTLKI SRVEAEDLGVYYCFQG SHVPFTFGSGTKLEIK RADAAPTVSIFPPSSE QLTSGGASVVCFLNNF YPKDINVKWKIDGSER QNGVLNSWTDQDSKDS TYSMSSTLTLTKDEYE RHNSYTCEATHKTSTS PIVKSFNRNEC |

The recombinant 301-17 IgG1 and IgG2a antibodies were tested and compared to the parent hybridoma-purified IgG3 antibody for binding characteristics as described below.

*301-17 IgG2a ProteOn Biosensor (BioRad) Binding to AbO:* Recombinant 301-17 IgG2a and hybridoma-purified 301-17 IgG3 were captured with anti-mouse IgG or amine coupling on Proteon GLM Sensor chips and tested for AbO binding (SynAging AbO). AbO 3 fold-dilutions were used: 1 uM, 0.33 uM, 0.11 uM, 37 nM, 12.3 nM . Assay buffer was PBS-E + Tween 20 + 2 mg/ml BSA.

**Results:**

Approximate kinetic values were:
Hybridoma:

$$KD = 26.9 \text{ nM}$$

IgG2a-301-17 antibody: KD = 16.2 - 19.5 nMNo binding was detected with control mouse IgG.

301-17 IgG2a *ProteOn Biosensor (BioRad) Binding to cyclic peptide epitope:* Recombinant 301-17 IgG2a was amine-coupled to Proteon GLH biosensor chip and tested for binding to cyclopeptide of SEQ ID NO: 2 coupled to BSA. Cyclo-BSA 3-fold dilutions were used from 9 nM to 111 pM. Assay buffer was PBS-E + 0.05% Tween + 10 mg/ml BSA. Antibody 301-17 IgG2a was found to bind cyclic peptide (SEQ ID NO: 12) conjugated to BSA with an approximate KD of 17 pM (average of 3 tests). No or negligible binding was detected for other commercial Abeta antibodies tested (pan-Abeta 6E10, Biolegend) and rabbit anti-Abeta antibodies (D54D2, Cell Signaling; ab201060, (abcam; NBP1-78007, Novus).

*301-17 IgG1 MAAS-2 binding to AbO:* Recombinant 301-17 IgG1 and hybridoma-purified 301-17 IgG3 were immobilized on MAAS-2 sensor chips and tested for binding to AbO (SynAging) at 1 uM. Under the conditions tested, the recombinant IgG1 301-17 antibody gave a greater signal than the hybridoma -purified antibody in 2 tests (40-55 BRU vs 15-25 BRU, respectively). Little or no binding was detected with control mouse IgG.

*301-17 IgG1 MAAS-2 binding to cyclicpeptide epitope:* Recombinant301-17 IgG1 was immobilized on MAAS-2 sensor chip and tested for binding to cyclopeptide of SEQ ID NO: 12 coupled to BSA at pH 6.5, 7.5 or 8.0. Equivalently high levels of binding were observed for 301-17 IgG1 under all 3 pH conditions (-400 BRUs). Little or no binding was detected under any of the pH conditions for control mouse IgG or the pan-Abeta 6E10 antibody (Biolegend)

**Example 6**

**Inhibition of Oligomer Propagation**

The biological functionality of antibodies was tested in vitro by examining their effects on Amyloid Beta (Aβ) aggregation using the Thioflavin T (ThT) binding assay. Aβ aggregation is induced by and propagated through nuclei of preformed small Aβ oligomers, and the complete process from monomeric Aβ to soluble oligomers to insoluble fibrils is accompanied by concomitantly increasing beta sheet formation. This can be monitored by ThT, a benzothiazole salt, whose excitation and emission maxima shifts from 385 to 450nm and from 445 to 482nm respectively when bound to beta sheet-rich structures and resulting in increased fluorescence. Briefly, Aβ 1-42 (Bachem Americas Inc., Torrance, CA) was solubilized, sonicated, diluted in Tris-EDTA buffer (pH7.4) and added to wells of a black 96-well microtitre plate (Greiner Bio-One, Monroe, NC) to which equal volumes of cyclopeptide raised antibody or irrelevant mouse IgG antibody isotype controls were added, resulting in a 1:5 molar ratio of Aβ1-42 peptide to antibody. ThT was added and plates incubated at room temperature for 24 hours, with ThT fluorescence measurements (excitation at 440nm, emission at 486nm) recorded every hour using a Wallac Victor3v 1420 Multilabel Counter (PerkinElmer, Waltham, MA). Fluorescent readings from background buffer were subtracted from all wells, and readings from antibody only wells were further subtracted from the corresponding wells.

Aβ42 aggregation, as monitored by ThT fluorescence, demonstrated a sigmoidal shape characterized by an initial lag phase with minimal fluorescence, an exponential phase with a rapid increase in fluorescence and finally a plateau phase during which the Aβ molecular species are at equilibrium and during which there is no increase in fluorescence. Co-incubation of Aβ42 with an irrelevant mouse antibody did not have any significant effect on the aggregation process. In contrast, co-incubation of Aβ42 with the test antibodies completely inhibited all phases of the aggregation process. Results obtained with antibody 301-11 are shown in FIG. 1.

Near identical results were obtained with 301-17 as well as 301-3.

As the ThT aggregation assay mimics the in vivo biophysical / biochemical stages of Aβ propagation and aggregation from monomers, oligomers, protofibrils and fibrils that is pivotal in AD pathogenesis, the antibodies demonstrate the potential to completely abrogate this process. Isotype control performed using mouse IgG control antibody showed no inhibition.

**Example 7**

**Toxicity inhibition assay**

The inhibition of toxicity of A-beta42 oligomers by antibodies can be tested in a rat primary cortical neuron assay.

Antibody and control IgG are each adjusted to a concentration such as 2 mg/mL. Various molar ratios of A-beta oligomer and antibody are tested along with a vehicle control, A-beta oligomer alone and a positive control such as the neuroprotective peptide humanin HNG.

An exemplary set up is shown in Table 9.

Following preincubation for 10 minutes at room temperature, the volume is adjusted to 840 microlitres with culture medium. The solution is incubated for 5 min at 37C. The solution is then added directly to the primary cortical neurons and cells are incubated for 24h. Cell viability can be determined using the MTT assay.

**Table 9**

| AβO / MAB molar ratio | AβO (μL) | AβO (μM) | AB (μM) | AB (μL) | Medium (μL) | Final volume (μL) |
|---|---|---|---|---|---|---|
| | | | | | | |
| 5/1 | 1.68 | 4.2 | 0.84 | 12.73 | 185.6 | 200 |
| 1/1 | 1.68 | 4.2 | 4.20 | 63.64 | 134.7 | 200 |
| 1/2 | 1.68 | 4.2 | 8.4 | 127.27 | 71.1 | 200 |

| AβO working solution: 2,2 mg/mL - 500 μM | |
|---|---|
| CTRL vehicle: | 1,68 μL of oligomer buffer + 127,3 μL PBS + 711 μL culture medium |
| CTRL AβO: | 1,68 μL of AβO + 127,3 μL PBS + 711 μL culture medium |
| | 1,68 μL of AβO + 8,4 μL HNG (100 nM final) + 127,3 μL PBS + 702,6 μL culture |
| CTRL HNG: | medium |

In the absence of A-beta oligomers, the 301-17 antibody alone had no effect on neuronal cell viability. When incubated in the presence of A-beta oligomers, the antibody inhibited A-beta oligomer-induced neuronal death at all molar ratios tested

**Example 8**

**In vivo toxicity inhibition assay**

The inhibition of toxicity of A-beta42 oligomers by the antibodies can be tested in vivo in mouse behavioral assays.

**Novel Object Recognition (NOR)**

The Novel Object Recognition (NOR) model utilizes the normal behavior of rodents to investigate novel objects for a significantly longer time than known objects. This test assesses recognition memory for items and its human equivalent is the visual pairwise-comparison (VPC). Recognition of objects is mediated by the perirhinal cortex in rodents, primates and humans. AD pathology develops first in the perirhinal and enthorinal cortex before the hippocampus. The VPC task detects memory deficit in mild cognitive impairment (MCI) and conversion from MCI to AD is predicted by this task (16).

Results:

The assay was performed by (SynAging SAS, Vandœuvre-lès-Nancy, France). Twelve C57BL6J mice per group (11-12 weeks old) were ICV-injected with vehicle (buffer used for Aβ oligomerization) or AβO (50 pmoles) in the presence of vehicle (PBS) or antibody 301-17 on day 0. The cognitive performance of all mice was determined by a Novel Object Recognition (NOR) test performed at days +7 and +8.

The study, done in blind to the operators, involved a total of 48 mice divided in four experimental groups with 12 mice per experimental group. All animals received a single (and unilateral) ICV injection of vehicle OR AβO in the absence or presence of antibody in a total volume of 5 μL. The experimental groups were defined as follow:

- GROUP A (vehicle CTRL): ICV injection of vehicle (n = 12)
- GROUP B (AβO CTRL): ICV injection of AβO (n = 12)
- GROUP C (Antibody CTRL): ICV injection of AβO + antibody (n = 12)
- GROUP D (Treatment): ICV injection of AβO + antibody (n = 12)

Before ICV injection, 4 μL of antibody 1 (*i.e.* 112 pmoles) were incubated for 30 minutes at room temperature with 1 μL vehicle (*i.e.* buffer for Aβ oligomerization) or 1 μL AβO (50 pmoles) corresponding to an antibody/AβO molar ratio of 2.24.

At day 0, mice received a single 5 μL ICV injection of vehicle or AβO in the presence of vehicle or antibody.

The NOR test was conducted in one trial with all 48 mice at days +7 and +8. One day before the cognitive test (*i.e.* at Day +7), mice are habituated during a 10 min trial during which they are placed in an empty open field. The day of the cognitive test (*i.e.* Day +8), animals are placed in the same open field and are allowed to explore freely two identical objects for a trial of five minutes (acquisition trial). Then the animals are returned to their home cage for an inter-trial time of five minutes. During the retention trial, animals are allowed to explore two different objects: the same familiar object and one novel object. During this time, the experimenter, blind to the treatment, records the time the mouse is <u>actively</u> exploring each object. All trials are video recorded (Smart v3.0 software, Bioseb). A discrimination index (DI) is then generated: (DI) = (time exploring novel object -time exploring familiar object) / total exploration time. If the total exploration time is ≤ 5 s, animals are excluded from the calculation of the discrimination index and statistical analysis.

Mice from the vehicle control group (Group A) exhibited normal behavior with a mean discrimination index of 0.443 ± 0.053. These results are in agreement with previous observations of similar control groups at SynAging. As expected, a single ICV injection of AβO (Group B) resulted in a significant impairment (p<0.0001) of the cognitive performance when compared to vehicle control mice; with a mean discrimination index of -0.062 ± 0.048. ApO-injected mice were not able to discriminate between novel and familiar objects.

Mice dosed with antibody in the presence of vehicle (Group C) were found to exhibit normal cognitive performances with a mean discrimination index of 0.439 ± 0.049. These mice were not significantly different from vehicle control mice (p=0.9163) and significantly different from AβO injected mice (p<0.0001).

When co-injected with AβO, the antibody fully prevented AβO-induced cognitive deficits in the NOR test. Indeed, mice from Group D exhibited a mean discrimination index of 0.481 ± 0.055, not different from control mice (p=0.6126) but different from AβO-injected mice (p=0.0002) . Taken together, the data suggest that antibody 301-17 offered protection against AβO-induced cognitive deficits.

### Synaptic Markers

In addition to behavioral assays, brain tissue can be collected and analyzed for levels of synaptic markers (PSD95, SNAP25, synaptophysin) and inflammation markers (IL-1-beta and TNF-alpha). Mice are sacrificed at ~14 days post-ICV injection of oligomers and perfused with saline. Hippocampi are collected, snap frozen and stored at -80°C until analyzed. Protein concentrations of homogenized samples are determined by BCA. Concentration of synaptic markers are determined using ELISA kits (Cloud-Clone Corp, USA). Typically, synaptic markers are reduced by 25-30% in mice injected with A-beta oligomers and restored to 90-100% by the humanin positive control. Concentrations of the IL-1-beta inflammatory markers are increased approximately 3-fold in mice injected with A-beta oligomers and this increase is largely prevented by humanin.

Brains are collected from mice that underwent the behavioral testing.

The hippocampus (relevant structure for memory formation) is dissected and homogenized in RIPA buffer containing an anti-protease cocktail. The tissue is lysed by 3 freeze thaw cycles carried out in liquid nitrogen and a water bath at 37C. and the supernatants are recovered after centrifuging.

The lysate can be analyzed for levels of TNF-alpha (increases with inflammation) and levels of the synaptic markers PSD-95 and SNAP-25 (which go down when there is synaptic damage).

The antibody showed complete protection in the behavioral assay. It is expected that brains will also show an improvement in both SNAP25 and PSD-95 levels and a decrease in TNF-alpha levels in the brain.

**Example 9**

**In vivo propagation inhibition assay**

In vivo propagation of A-beta toxic oligomers and associated pathology can be studied in various rodent models of Alzheimer's disease (AD). For example, mice transgenic for human APP (e.g. APP23 mice) or human APP and PSEN1 (APPPS1 mice) express elevated levels of A-beta and exhibit gradual amyloid deposition with age accompanied by inflammation and neuronal damage. Intracerebral inoculation of oligomer-containing brain extracts can significantly accelerate this process (13, 14). These models provide a system to study inhibition of A-beta oligomer propagation by test antibodies administered intracerebrally or systemically.

**Table 10 A-beta Sequences and compounds**

1)
HHQK (SEQ ID NO: 7)
CGHHQKG, cyclo(CGHHQKG) (SEQ ID NO: 12)

**Table 11**

Human A-beta 1-42
DAEFRHDSGYEVHHQKLVFFAEDVGSNKGAIIGLMVGGVVIA (SEQ ID NO: 73)

**Example 12**

**Recombinant antibodies (HHQK)** Recombinant IgG1 and IgG2a 301-17 constructs were made by grafting the variable region of hybridoma-derived 301-17 onto a murine IgG1 or IgG2a backbone (WuXi, Biologics).

The 301-17 IgG1 and IgG2a antibodies were tested and compared to the parent hybridoma-purified IgG3 antibody for binding characteristics as described below.

*301-17 IgG2a ProteOn Biosensor (BioRad) Binding to AbO:* Recombinant 301-17 IgG2a and hybridoma-purified 301-17 IgG3 were captured with anti-mouse IgG or amine coupling on Proteon GLM Sensor chips and tested for AbO binding (SynAging AbO). AbO 3 fold-dilutions were used: 1 uM, 0.33 uM, 0.11 uM, 37 nM, 12.3 nM . Assay buffer was PBS-E + Tween 20 + 2 mg/ml BSA.

**Results:**

Approximate kinetic values were:

Hybridoma: KD = 26.9 nM

IgG2a-301-17 antibody: KD = 16.2 - 19.5 nM

No binding was detected with control mouse IgG.

Recombinant 301-17 IgG1had a similar KD to the IgG2a recombinant.

301-17 IgG2a *ProteOn Biosensor (BioRad) Binding to cyclic peptide epitope:* Recombinant 301-17 IgG2a was amine-coupled to Proteon GLH biosensor chip and tested for binding to cyclopeptide of SEQ ID NO: 2 coupled to BSA. Cyclo-BSA 3-fold dilutions were used from 9 nM to 111 pM. Assay buffer was PBS-E + 0.05% Tween + 10 mg/ml BSA. Antibody 301-17 IgG2a was found to bind cyclic peptide (SEQ ID NO: 2) conjugated to BSA with an approximate KD of 17 pM (average of 3 tests). No or negligible binding was detected for other commercial A-beta antibodies tested (pan-Abeta 6E10, Biolegend) and rabbit anti-A-beta antibodies (D54D2, Cell Signaling; ab201060, (abcam; NBP1-78007, Novus).

*301-17 IgG1 MAAS-2 binding to AbO:* Recombinant 301-17 IgG1 and hybridoma-purified 301-17 IgG3 were immobilized on MAAS-2 sensor chips and tested for binding to AbO (SynAging) at 1 uM. Under the conditions tested, the recombinant IgG1 301-17 antibody gave a greater signal than the hybridoma -purified antibody in 2 tests (40-55 BRU vs 15-25 BRU, respectively). Little or no binding was detected with control mouse IgG.

*301-17 IgG1 MAAS-2 binding to cyclic peptide epitope:* Recombinant 301-17 IgG1 was immobilized on MAAS-2 sensor chip and tested for binding to cyclopeptide of SEQ ID NO: 2 coupled to BSA at pH 6.5, 7.5 or 8.0. Equivalently high levels of binding were observed for 301-17 IgG1 under all 3 pH conditions (-400 BRUs). Little or no binding was detected under any of the pH conditions for control mouse IgG or the pan-Abeta 6E10 antibody (Biolegend)

## Example 13

Humanized antibody binding in soluble brain extracts

## Methods

Pool of soluble brain extracts injected at 0.5ml/min through Superdex 75(10/300) HPLC column for 50 minutes and 0.25ml fractions collected:

Soluble human AD brain extracts were separated into LMW (<70kDa) and high molecular (HMW; >70kDa) fractions by size-exclusion chromatography (SEC). Antibody binding to SEC fractions was assessed by surface plasmon resonance (SPR).

Specifically, pooled fractions were concentrated and total protein concentration determined by BCA assay. Pooled fractions were diluted to 100ug/ml and injected over immobilized antibodies on sensor chip of MASS2.

## Results

SEC fractionation of soluble human AD brain extracts gave rise to a reproducible pattern with LMW peaks consistent with the presence of reportedly toxic dimers, tetramers and dodecamers. Humanized antibody of 301-17 (VH2 Vk5) (SEQ ID NO: 45, 46 and SEQ ID NO: 65, 66) was compared to the mouse monoclonal version (301-17; Table 3) and other A-beta antibodies.

As shown in FIG. 2, mouse monoclonal 301-17 and aducanumab show equivalent binding to the toxic oligomer-enriched LMW fraction of soluble human AD brain extract.

SPR analysis showed preferential binding of humanized 301-17 to the toxic oligomer-enriched LMW fraction compared to aducanumab and bapineuzumab. The binding response of humanized 301-17 for the LMW fraction was -1.5-2 fold greater than that obtained with aducanumab.

## Example 14

**Methods:** Frozen human AD brain sections were stained with humanized 301-17 and other Abeta-directed antibodies to evaluate the degree of binding to parenchymal Abeta plaque and vascular A beta deposits.

**Results:** Clear binding of parenchymal and vascular Abeta in AD brain was observed with aducanumab and bapineuzumab, consistent with the clinical occurrence of ARIA associated with these antibodies. By comparison, no immunoreactivity of Abeta deposits was observed with humanized 301-17 (VH2Vk5).

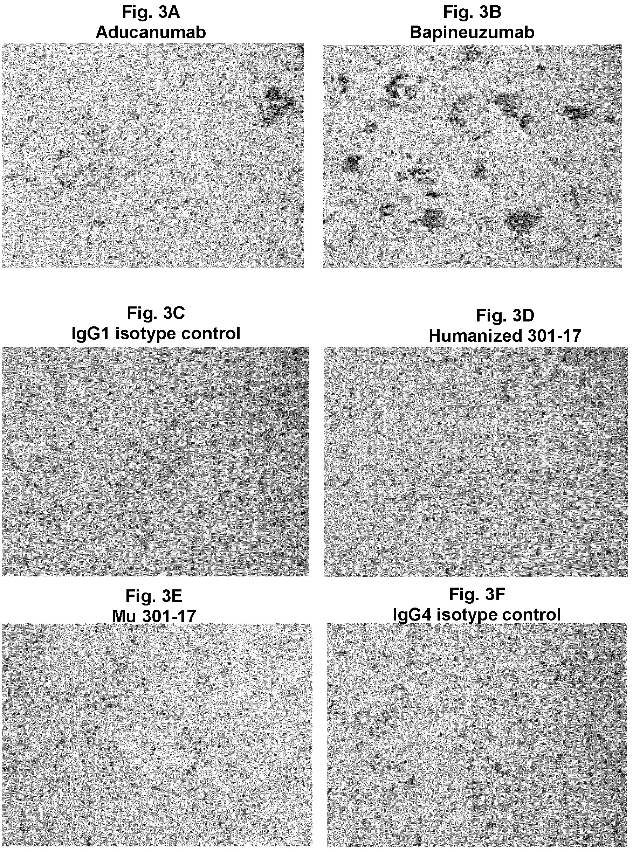

**Conclusions:** Results obtained with AD patient material (Ex 13 and 14) suggest that humanized 301-17 may achieve greater therapeutic potency compared to other AD-directed antibodies due to: 1) Selective targeting of soluble toxic LMW oligomers, and 2) Reduced risk of ARIA allowing for safe administration of higher doses of antibody. This data is shown in Fig. 3

## Example 15

A$\beta$ monomers and oligomers

Recombinant A$\beta$42 peptide (California Peptide, Salt Lake City UT, USA) was dissolved in ice-cold hexafluoroisopropanol (HFIP). The HFIP was removed by evaporation overnight and dried in a SpeedVac centrifuge. To prepare monomers, the peptide film was reconstituted in DMSO to 5mM, diluted further to 100$\mu$M in distilled water (dH2O) and used immediately. Oligomers were prepared by diluting the 5mM DMSO peptide solution in phenol red-free F12 medium (Life Technologies Inc., Burlington ON, Canada) to a final concentration of 100$\mu$M and incubated for 24 hours at 4°C followed by immediate use or storage at -80C.

Brain extract

Brain tissues from 11 different human AD patients were obtained from the University of Maryland Brain and Tissue Bank and from Dr. Jiri Safar at Case Western Reserve University (Cleveland, Ohio, USA). The clinical diagnosis of AD was based on NINCDS-ADRDA criteria. Samples from frontal cortex were weighed and subsequently submersed in a volume of fresh, ice cold TBS buffer and EDTA-free protease inhibitor cocktail from Roche Diagnostics (Laval QC, Canada) such that the final concentration of brain tissue was 20% (w/v). Tissue was homogenized in this buffer using a mechanical probe homogenizer (3 × 30 sec pulses with 30 sec pauses in between, all performed on ice). TBS-homogenized samples were then subjected to ultracentrifugation for 90 min. Supernatants (soluble extracts) were collected, aliquoted and stored at -80°C. The protein concentration was determined using a bicinchoninic acid (BCA) protein assay. Pools of brain extracts from 3-8 patients were used in each analysis.

**Size exclusion chromatography**

Pooled soluble brain extracts were injected at 0.5 ml/min through a Superdex 75 (10/300) HPLC column for 50 minutes and 0.25ml fractions were collected. Molecular weight (MW) markers were run separately. Protein peaks were monitored by absorbance at O.D. 280 nm. Fractions corresponding to a MW of ~8kDa to ~70kDa were pooled into a low molecular weight (LMW) fraction. Aβ monomers (MW ~4.5kDa) were excluded from the LMW fraction. Fractions corresponding to a MW of >70kDa to ~700kDa were pooled into a high molecular weight (HMW) fraction. The LMW and HMW fractions were concentrated and total protein concentration was determined in a BCA assay. The fractions were then diluted in PBS-EP, BSA (2mg/ml) buffer to 100 μg /ml for surface plasmon resonance (SPR) analysis.

**Measurement of total Aβ and aggregated Aβ in brain extract**

The amount of aggregated Aβ and total Aβ (monomers and aggregates) in the LMW and HMW fractions of pooled human AD soluble brain extract (pool from 3 brains) were measured at QPS (Grambach, Austria). Total amounts of Aβ38, 40 and 42 were determined in a commercial immunosorbent assay (MSD, Rockville MD, USA) using peptide standards. Aggregated Aβ levels were measured using the Amorfix Aggregated Aβ Assay (A4). Briefly, aggregated Aβ was separated from monomeric Aβ via the matrix of the Amorfix Disaggregation Plate. While oligomers attach to the matrix, monomers are found in the flow through. After two washing steps, the attached Aβ aggregates are monomerized and eluted with HFIP. Monomerized Aβ in the HFIP eluate was dried under a fume hood until complete evaporation of HFIP and resuspended in assay buffer. Measurement of resolved monomerized Aβ aggregates was carried out in the MSD assay for Aβ38, 40 and 42. The total protein concentration in the LMW and HMW brain fractions was measured by BCA and the results are expressed as pg Aβ species per mg total protein.

**Surface plasmon resonance analysis**

Surface plasmon resonance measurements were performed using a Molecular Affinity Screening System (Sierra Sensors GmbH, Hamburg, Germany). Purified antibodies were immobilized on sensorchips . Preparations of Aβ peptide monomers, synthetic Aβ oligomers or pooled soluble human AD brain extracts (100 μg/ml) were injected over the surfaces followed by a dissociation phase. Sensorgrams were double-reference subtracted. The SPR results shown were replicated in 2-8 independent studies.

**Immunohistochemistry**

Fresh frozen AD brain sections were exposed to antigen retrieval citrate buffer (Target Retrieval Solution, Dako, Santa Clara CA, USA) for 20 min and incubated in a humidified chamber with serum-free protein blocking reagent (Dako) for 1h to block non-specific staining. The sections were incubated overnight at 4°C with primary antibodies (mu301-17, hu 301-17, aducanumab, bapineuzumab, isotype controls) at 1 μg/ml and washed 3 times for 5 min in Tris-buffered saline containing 0.1% TritonX-100 (TBS-T) buffer. Secondary HRP-conjugated rabbit anti-human IgG (0.4 μg/ml; Abcam, San Francisco CA, USA) or sheep anti-mouse IgG (1:1000 dilution; GE Healthcare, Chicago IL, USA) antibodies were added to the sections and incubated for 1 hour, followed by 3 washes in TBS-T buffer. Secondary antibody was also added to sections that were not exposed to primary antibody as a negative control. The HRP enzyme substrate, biaminobezidine (DAB) chromogen reagent (Vector Laboratories, Burlingame CA, USA), was then added to the sections followed by rinsing with distilled water. The sections were counterstained with haematoxylin QS (Vector Laboratories, Burlingame CA, USA). The slides were examined under a light microscope (Zeiss Axiovert 200M, Carl Zeiss Toronto ON, Canada) and representative images were captured using a Leica DC300 digital camera and software (Leica Microsystems Canada Inc., Vaughan ON, Canada). Images shown are representative of results obtained with 3 different AD brains.

**Central nervous system exposure**

Aged APP/PS1 (APPswe/PSEN1dE9 ) mice and wild type (WT) littermates (54-71 weeks old) were injected intraperitoneally (i.p.) with 30 mg/kg hu 301-17, aducanumab or vehicle (PBS) as a negative control. Plasma was collected prior to treatment and on days 1, 7, 14 and 21 for assessment of circulating levels of human IgG. Animals were sacrificed immediately after plasma collection and PBS perfusion. Brains were then collected and flash frozen. Brains and plasma were stored at -80C until use. Brain homogenates (10% w/v) were generated in RIPA buffer with an Omni tissue homogenizer (Omni International, Inc. Kennesaw, GA, USA), at half power for 30 sec, three times. Homogenates were then sonicated for 15 sec at half power, followed by clearance of debris by centrifugation (2,000xg). Levels of human IgG in plasma (1:10,000 dilution) and brain homogenates (1:5 dilution) from individual mice were measured using the Human IgG Immunotek ELISA (Zeptomatrix, Buffalo NY, USA) according to manufacturer's instructions. Results are expressed as mean µg/ml (plasma) or ng/g (brain) ±SEM

## Results

**Comparison of humanized 301-17 binding profile to other Aβ-directed antibodies**

The IgG4 isotype (S241P hinge mutation) humanized antibody VH2Vκ5was compared to other antibodies. The humanized antibody retained selective binding for synthetic AβO vs Aβ monomers as assessed by SPR as well as lack of plaque binding by immunohistochemistry on frozen AD brain sections. As shown in Fig. 3, the Aβ-directed antibodies bapineuzumab and aducanumab, known to bind fibrils, showed robust staining of parenchymal Aβ plaque and vascular deposits while neither the parent monoclonal (mu 301-17) nor the humanized 301-17 showed any staining above background. Similar results were obtained with brain sections from aged transgenic APP/PS1 transgenic mice). Binding to plaque and vascular deposits in AD clinical trials has been associated with the induction of amyloid-related imaging abnormalities due to edema (ARIA-E) and micro hemorrhages (ARIA-H).

Binding of hu301-17 to low molecular weight toxic Aβ oligomer-enriched soluble AD brain extracts

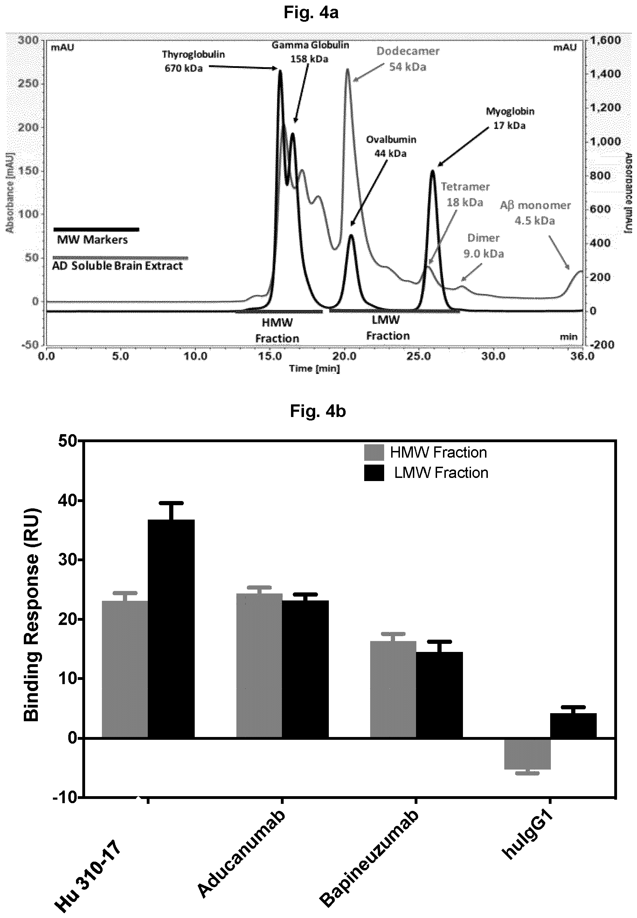

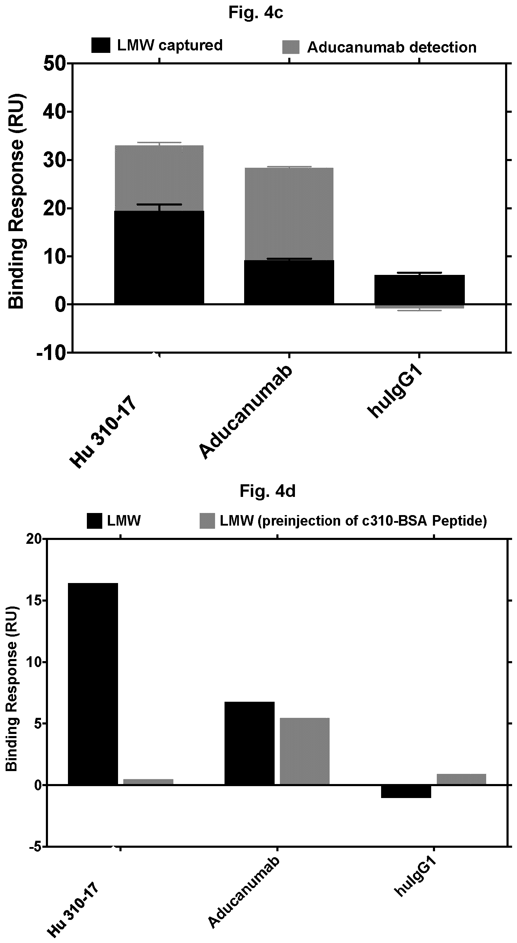

Examination of soluble Aβ species in AD brain extracts by several investigators has indicated that the neurotoxic activity resides primarily in the low molecular weight (LMW) fraction of AβO (dimers, trimers, tetramers, dodecamers) while high molecular weight (HMW) aggregates are largely inert though they reportedly can dissociate into LMW species . Therefore, size exclusion chromatography (SEC) of pooled soluble extracts from AD brains was performed . SEC fractionation of soluble AD brain extract gave rise to a highly reproducible pattern with protein peaks in the MW regions expected to contain LMW AβO (Fig 4a). Fractions corresponding to ~8 -70 kDa were pooled into a LMW fraction expected to contain AβO in the dimer to dodecamer range and excluding monomers. Fractions corresponding to >70-700 kDa were pooled into a HMW fraction. Measurements of total Aβ38, 40, 42 (MSD assay) showed all 3 species to be present in both the LMW and HMW fractions with Aβ42 representing the major aggregated Aβ species (A4 assay).
Binding of immobilized hu301-17 and other Aβ-directed antibodies to the LMW and HMW fractions of soluble AD brain extract was assessed by SPR. Representative results from several separate assays utilizing different pooled extracts derived from multiple AD brains are shown in Fig. 4b. The hu301-17 antibody consistently showed high and preferential binding to the LMW fraction. By comparison, aducanumab and bapineuzumab showed lower and indiscriminate binding of the LMW vs HMW fraction.
Aβ protein and Aβ aggregates were determined to be present in the LMW fraction but to rule out the possibility that hu301-17 may have been binding to other protein(s) also present in this fraction, a sandwich SPR assay was conducted whereby the material captured by immobilized hu301-17 was subsequently exposed to a detector antibody. Aducanumab was chosen as the detector antibody as it is known to be specific for Aβ and was expected to bind/detect material captured by immobilized aducanumab, thereby acting as a positive control. As illustrated in Fig. 4c, aducanumab detection in the sandwich assay did produce a signal against material captured by either hu301-17 or aducanumab, thereby confirming the binding of hu 301-17 to AβO in the LMW fraction. The material giving rise to the low signal observed after capture with control human IgG in this assay was not detected by aducanumab consistent with a low degree of non-specific background binding.
The specific nature of hu301-17 binding was further demonstrated in an SPR assay showing that pre-exposure of immobilized hu301-17 to its cognate cyclic peptide epitope completely prevented subsequent binding to the LMW fraction (Fig. 4d). By comparison, pre-exposure of immobilized aducanumab to the cyclic peptide epitope had no appreciable impact on its ability to subsequently bind the LMW fraction since aducanumab recognizes a different Aβ epitope. Taken together, these results suggest that hu301-17, in addition to being selective for AβO vs monomers and plaque, also exhibits superior targeting of the LMW toxic oligomer-enriched fraction of AD brain extract compared to other Aβ-directed antibodies.

**CNS exposure to hu301-17 delivered systemically**

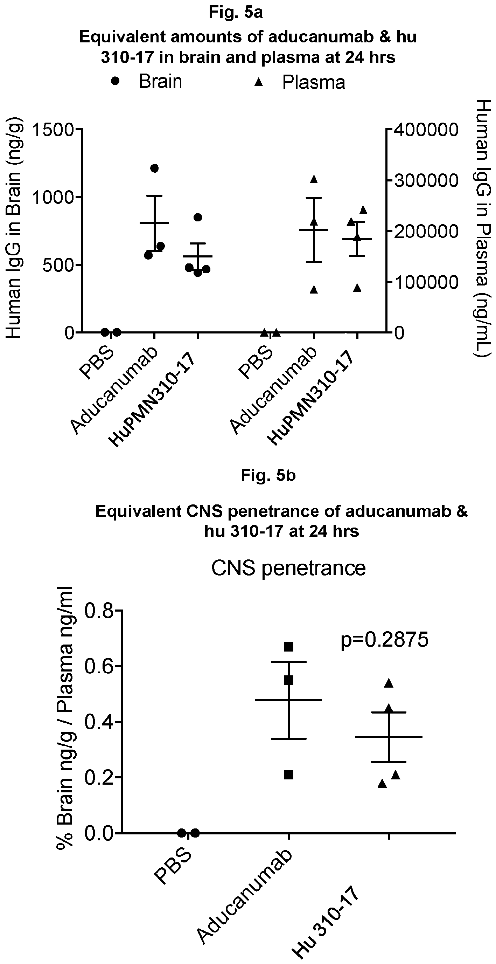

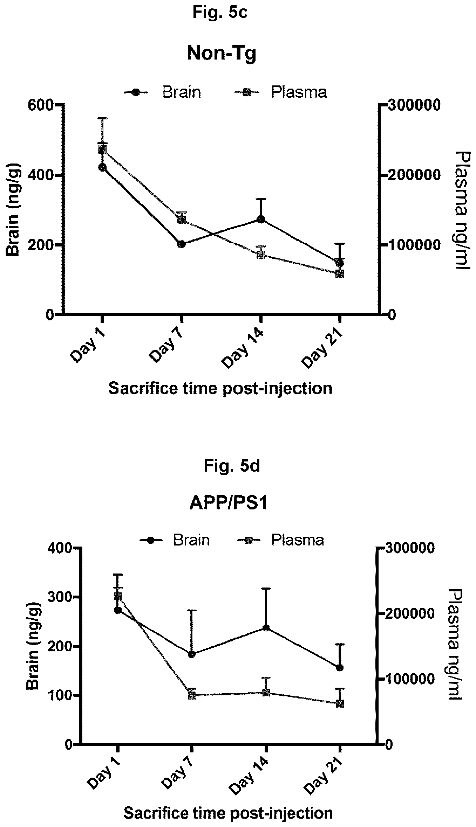

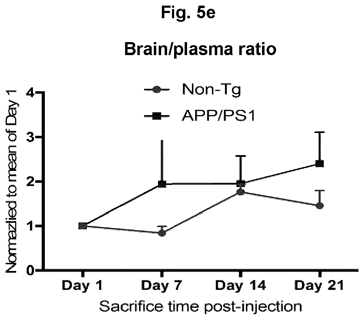

The ability of hu301-17 to cross the blood brain barrier (BBB) and enter the central nervous system (CNS) from the periphery was assessed and compared to that of aducanumab in aged wild type mice (15-17 months old). Mice were dosed with a single intraperitoneal (i.p.) injection of 30 mg/kg antibody and levels of human IgG present in the plasma and perfused brains were measured 24 hr later by ELISA. As shown in Fig 5, equivalent amounts of hu301-17 and aducanumab were detected in plasma and brain (Fig. 5a) demonstrating a comparable degree of CNS penetrance (Fig. 5b) in the range of -0.3% as previously reported for aducanumab [18]. As expected, no human IgG was detected in mice injected with PBS alone as a negative control (Fig 5a).

Additional kinetic assessment of hu301-17 was conducted in aged (13-17 months old) transgenic APP/PS1 mice and wild type littermates. Plasma and brain levels of human IgG were measured on days 1, 7, 14 and 21 after i.p. administration of 30 mg/kg. In spite of declining plasma levels over time, detectable levels of hu301-17 in the brain were still measurable out to day 21 (Fig 6a,b). Interestingly, APP/PS1 mice showed a trend for greater relative retention of hu301-17 in CNS vs plasma over time, consistent with engagement of target AβO present in the brain of transgenic mice but not in the wild type littermates (Fig 6c). These results indicate that the CNS penetrance and pharmacokinetic properties of hu301-17 are comparable to those of other monoclonal antibodies against Aβ targets.

CITATIONS FOR REFERENCES REFERRED TO IN THE SPECIFICATION

[1] Gabriela A. N. Crespi, Stefan J. Hermans, Michael W. Parker, and Luke A. Miles. Molecular basis for mid-region amyloid-b capture by leading Alzheimer's disease immunotherapies SCIENTIFIC REPORTS | 5: 9649, 2015| DOI: 10.1038/srep09649

[2] Vincent J. Hilser and Ernesto Freire. Structure-based calculation of the equilibrium folding pathway of proteins. correlation with hydrogen exchange protection factors. J. Mol. Biol.,262.756-772, 1996. The COREX approach.

[3] Samuel I. A. Cohen, et. al. Proliferation of amyloid-β42 aggregates occurs through a secondary nucleation mechanism. Proc. Natl.l Acad. Sci. USA, 110(24):9758-9763, 2013.

[4] Pietro Sormanni, Francesco A. Aprile, and Michele Vendruscolo. The camsol method of rational design of protein mutants with enhanced solubility. J of Mol Biol, 427(2):478-490, 2015.

[5] Deborah Blacker, MD, ScD; Marilyn S. Albert, PhD; Susan S. Bassett, PhD; Rodney C. P. Go, PhD; Lindy E. Harrell, MD, PhD; Marshai F. Folstein, MD Reliability and Validity of NINCDS-ADRDA Criteria for Alzheimer's Disease The National Institute of Mental Health Genetics Initiative. Arch Neurol. 1994;51(12):1198-1204. doi:10.1001/archneur.1994.00540240042014.

[6] Hamley, I.W. PEG-Peptide Conjugates 2014; 15, 1543-1559; dx.doi.org/10.1021/bm500246w

[7] Roberts, MJ et al Chemistry for peptide and protein PEGylation 64: 116-127.

[8] J.X.Lu, W.Qiang, W.M.Yau, C.D.Schwieters, S.C.Meredith, R.Tycko, MOLECULAR STRUCTURE OF BETA-AMYLOID FIBRILS IN AD BRAIN TISSUE. CELL Vol. 154 p.1257 (2013)

[9] Y.Xiao, B.MA, D.McElheny, S.Parthasarathy, F.Long, M.Hoshi, R.Nussinov, Y.Ishii, A BETA (1-42) FIBRIL STRUCTURE ILLUMINATES SELF-RECOGNITION AND REPLICATION OF AMYLOID IN ALZHEIMER'S DISEASE. NAT.STRUCT.MOL.BIOL. Vol. 22 p.499 (2015).

[10] A.Petkova,W.Yau,R.Tycko EXPERIMENTAL CONSTRAINTS ON QUATERNARY STRUCTURE IN ALZHEIMER'S BETA-AMYLOID FIBRILS BIOCHEMISTRY V. 45 498 2006.

[11] Giulian D, Haverkamp LJ, Yu J, Karshin W, Tom D, Li J, Kazanskaia A, Kirkpatrick J, Roher AE. The HHQK domain of β-amyloid provides a structural basis for the immunopathology of Alzheimer's disease, J.BiolChem. 1998, 273(45), 29719-26.

[12] Winkler K, Scharnagl H, Tisljar U, Hoschützky H, Friedrich I, Hoffmann MM, Hüttinger M, Wieland H, März W. Competition of Aβ amyloid peptide and apolipoprotein E for receptor-mediated endocytosis. J.Lipid Res.1999, 40(3), 447-55.

[13] SCIENTIFIC REPORTS | 5 : 9649 | DOI: 10.1038/srep09649].

[14] Yu YZ, Wang WB, Chao A, Chang Q, Liu S, Zhao M, et al. Strikingly reduced amyloid burden and improved behavioral performance in Alzheimer's disease mice immunized with recombinant chimeric vaccines by hexavalent foldable Ab 1-15 fused to toxin-derived carrier proteins. J Alzheimer's Dis 2014;41:243-60.

[15] Wang, HC; Yu, YZ; Liu, S; Zhao, M and Q Xu, Peripherally administered sera antibodies recognizing amyloid-_oligomers mitigate Alzheimer's disease-like pathology and cognitive decline in aged 3× Tg-AD mice, Vaccine 2016.

[16] Zola SM, Manzanares CM, Clopton P, Lah JJ, Levey AI. A behavioral task predicts conversion to mild cognitive impairment and Alzheimer's disease. Am J Alzheimer's Dis & other dementia. 2013, 28(2), 179-184.

[17] Peters SJ et al. Engineering an Improved IgG4 Molecule with Reduced Disulfide Bond Heterogeneity and Increased Fab Domain Thermal Stability, The Journal of Biological Chemistry (2012) 287, 24525-24533.

[18] Sevigny J et al (2016) The antibody aducanumab reduces AD plaque in Alzheimer's disease. Nature 537: 50-56

**Claims**

1. A humanized antibody comprising a heavy chain variable region and a light chain variable region, wherein:

   the heavy chain variable region comprises an amino acid sequence as set forth in SEQ ID NO: 46;
   the light chain variable region comprises an amino acid sequence as set forth in SEQ ID NO: 66; and
   the humanized antibody binds amyloid beta oligomers.

2. The humanized antibody of claim 1, wherein the heavy chain variable region amino acid sequence is encoded by a nucleotide sequence as set forth in SEQ ID NO: 45; or a codon degenerate or optimized version thereof; and the light chain variable region amino acid sequence is encoded by a nucleotide sequence as set out in SEQ ID NO: 65 or a codon degenerate or optimized version thereof.

3. The humanized antibody of any one of claims 1 to 2, wherein the humanized antibody is an IgG1, IgG2, IgG3 or IgG4 antibody or an antibody binding fragment, wherein said antibody binding fragment is selected from Fab, Fab', F(ab')2, scFv, dsFv, ds-scFv, dimers, minibodies, diabodies, and multimers thereof, preferably wherein the antibody binding fragment is a Fab fragment.

4. The humanized antibody of any one of claims 1 to 3, comprising the CH1 and/or CL sequence or a part thereof of IgG4, preferably wherein the CH1 and/or CL sequence comprises SEQ ID NO: 70 or 72 or a part thereof, or a conservative variant thereof or a sequence with at least 50%, 60%, 70%, 89%, 90% or 95% sequence identity to SEQ ID NO: 70 and/or 72, preferably wherein the humanized antibody comprises SEQ ID NO: 70 and/or 72, and/or CH1 and CH2 of SEQ ID NO: 70 or a conservative variant of any of the foregoing or a sequence with at least 50%, 60%, 70%, 89%, 90% or 95% sequence identity to any of the foregoing.

5. The humanized antibody of any one of claims 1 to 4, wherein the humanized antibody is a single chain antibody.

6. An immunoconjugate comprising the humanized antibody of any one of claims 1 to 5 and a detectable label or cytotoxic agent.

7. The humanized antibody of any one of claims 1 to 5 or the immunoconjugate of claim 6, for use in treating Alzheimer's disease, Lewy body dementia and/or cerebral amyloid angiopathy.

8. A composition comprising the humanized antibody of any one of claims 1 to 5 or the immunoconjugate of claim 6 and a diluent.

9. A nucleic acid molecule encoding the humanized antibody of any one of claims 1 to 5, preferably wherein the nucleic acid is comprised in a vector.

10. A cell expressing the humanized antibody of any one of claims 1 to 5.

11. The immunoconjugate of claim 6 for use in measuring a level of amyloid beta in vivo, in a subject at risk or suspected of having or having Alzheimer's disease, wherein the antibody is conjugated to a detectable label, preferably wherein the label is a positron emitting radionuclide.

12. An effective amount of a pharmaceutical composition comprising the humanized antibody or immunoconjugate of any one of claims 1 to 5 or 6, for use in treating Alzheimer's disease, Lewy body dementia and/or cerebral amyloid angiopathy in a subject in need thereof.

13. An effective amount of the humanized antibody or immunoconjugate of any one of claims 1 to 5 or 6, or a pharmaceutical composition for use according to claim 12, wherein the humanized antibody or immunoconjugate is administered directly to the brain or other portion of the CNS.

14. Use of the humanized antibody or immunoconjugate of any one of claims 1 to 5 or 6, or a pharmaceutical composition comprising said humanized antibody or immunoconjugate, in assessing the presence or levels of amyloid beta in a biological sample from a subject to be treated with the humanized antibody, immunoconjugate or pharmaceutical composition thereof according to claims 1 to 5 or 6.

**Patentansprüche**

1. Humanisierter Antikörper, der eine variable Region einer schweren Kette und eine variable Region einer leichten Kette umfasst, wobei:

   die variable Region der schweren Kette eine Aminosäuresequenz wie dargelegt in SEQ ID NO: 46 umfasst;
   die variable Region der leichten Kette eine Aminosäuresequenz wie dargelegt in SEQ ID NO: 66 umfasst; und
   der humanisierte Antikörper Amyloid-Beta-Oligomere bindet.

2. Humanisierter Antikörper nach Anspruch 1, wobei die Aminosäuresequenz der variablen Region der schweren Kette durch eine Nukleotidsequenz wie dargelegt in SEQ ID NO: 45 kodiert ist; oder eine Codon-degenerierte oder optimierte Version davon; und die Aminosäuresequenz der variablen Region der leichten Kette durch eine Nukleotidsequenz wie dargelegt in SEQ ID NO: 65 kodiert oder eine Codon-degenerierte oder optimierte Version davon ist.

3. Humanisierter Antikörper nach einem der Ansprüche 1 bis 2, wobei der humanisierte Antikörper ein IgG1-, IgG2-, IgG3- oder IgG4-Antikörper oder ein Antikörperbindungsfragment ist, wobei das Antikörperbindungsfragment ausgewählt ist aus Fab, Fab', F(ab')2, scFv, dsFv, ds-scFv, Dimeren, Minibodies, Diabodies und Multimeren davon, wobei bevorzugt das Antikörperbindungsfragment ein Fab-Fragment ist.

4. Humanisierter Antikörper nach einem der Ansprüche 1 bis 3, umfassend die CH1- und/oder CL-Sequenz oder einen Teil davon von IgG4, wobei bevorzugt die CH1- und/oder CL-Sequenz SEQ ID NO: 70 oder 72 oder einen Teil davon oder eine konservative Variante davon oder eine Sequenz mit zumindest 50%, 60 %, 70 %, 89 %, 90 % oder 95 % Sequenzidentität mit SEQ ID NO: 70 und/oder 72 umfasst, wobei bevorzugt der humanisierte Antikörper SEQ ID NO: 70 und/oder 72 und/oder CH1 und CH2 von SEQ ID NO: 70 oder eine konservative Variante von einem beliebigen des Vorstehenden oder eine Sequenz mit zumindest 50 %, 60 %, 70 %, 89 %, 90 % oder 95 % Sequenzidentität mit einem beliebigen des Vorstehenden umfasst.

5. Humanisierter Antikörper nach einem der Ansprüche 1 bis 4, wobei der humanisierte Antikörper ein Einzelkettenantikörper ist.

6. Immunkonjugat, das den humanisierten Antikörper nach einem der Ansprüche 1 bis 5 und eine nachweisbare Markierung oder ein zytotoxisches Mittel umfasst.

7. Humanisierter Antikörper nach einem der Ansprüche 1 bis 5 oder Immunkonjugat nach Anspruch 6 zur Verwendung beim Behandeln von Alzheimer-Krankheit, Lewy-Körperchen-Demenz und/oder zerebraler Amyloid-Angiopathie.

8. Zusammensetzung, umfassend den humanisierten Antikörper nach einem der Ansprüche 1 bis 5 oder das Immunkonjugat nach Anspruch 6 und ein Verdünnungsmittel.

9. Nukleinsäuremolekül, das den humanisierten Antikörper nach einem der Ansprüche 1 bis 5 kodiert, wobei bevorzugt die Nukleinsäure in einem Vektor umfasst ist.

10. Zelle, die den humanisierten Antikörper nach einem der Ansprüche 1 bis 5 exprimiert.

11. Immunkonjugat nach Anspruch 6 zur Verwendung beim Messen eines Spiegels von Amyloid Beta in vivo, bei einem Subjekt, bei dem das Risiko oder der Verdacht besteht, Alzheimer-Krankheit aufzuweisen, wobei der Antikörper an eine nachweisbare Markierung konjugiert ist, wobei bevorzugt die Markierung ein Positronen emittierendes Radionuklid ist.

12. Wirksame Menge einer pharmazeutischen Zusammensetzung, die den humanisierten Antikörper oder das Immunkonjugat nach einem der Ansprüche 1 bis 5 oder 6 umfasst, zur Verwendung beim Behandeln von Alzheimer-Krankheit, Lewy-Körperchen-Demenz und/oder zerebraler Amyloid-Angiopathie bei einem Subjekt, das dessen bedarf.

13. Wirksame Menge des humanisierten Antikörpers oder Immunkonjugats nach einem der Ansprüche 1 bis 5 oder 6 oder einer pharmazeutischen Zusammensetzung zur Verwendung nach Anspruch 12, wobei der humanisierte Antikörper oder das Immunkonjugat direkt an das Gehirn oder einen anderen Teil des ZNS verabreicht wird.

**14.** Verwendung des humanisierten Antikörpers oder Immunkonjugats nach einem der Ansprüche 1 bis 5 oder 6 oder einer pharmazeutischen Zusammensetzung, die den humanisierten Antikörper oder das Immunkonjugat umfasst, beim Beurteilen des Vorhandenseins oder der Spiegel von Amyloid Beta in einer biologischen Probe von einem Subjekt, das mit dem humanisierten Antikörper, dem Immunkonjugat oder der pharmazeutischen Zusammensetzung davon nach Anspruch 1 bis 5 oder 6 zu behandeln ist.

**Revendications**

**1.** Anticorps humanisé comprenant une région variable de chaîne lourde et une région variable de chaîne légère :

ladite région variable de chaîne lourde comprenant une séquence d'acides aminés telle que décrite dans SEQ ID n° : 46 ;
ladite région variable de chaîne légère comprenant une séquence d'acides aminés telle que décrite dans SEQ ID n° : 66 ; et
ledit anticorps humanisé se liant à des oligomères de bêta-amyloïde.

**2.** Anticorps humanisé selon la revendication 1, ladite séquence d'acides aminés de région variable de chaîne lourde étant codée par une séquence nucléotidique telle que décrite dans SEQ ID n° : 45 ; ou une dégénérescence de codon ou une version optimisée de celle-ci ; et ladite séquence d'acides aminés de région variable de chaîne légère étant codée par une séquence nucléotidique telle que décrite dans SEQ ID n° : 65 ; ou une dégénérescence de codon ou une version optimisée de celle-ci.

**3.** Anticorps humanisé selon l'une quelconque des revendications 1 à 2, ledit anticorps humanisé étant un anticorps IgG1, IgG2, IgG3 ou IgG4 ou un fragment de liaison d'anticorps, ledit fragment de liaison d'anticorps étant choisi parmi Fab, Fab', F(ab')2, scFv, dsFv, ds-scFv, les dimères, les mini-corps, les dianticorps et les multimères de ceux-ci, de préférence ledit fragment de liaison d'anticorps étant un fragment Fab.

**4.** Anticorps humanisé selon l'une quelconque des revendications 1 à 3, comprenant la séquence CH1 et/ou CL ou une partie de celle-ci d'IgG4, de préférence ladite séquence CH1 et/ou CL comprenant la SEQ ID n° : 70 ou 72 ou une partie de celle-ci, ou un variant conservateur de celle-ci ou une séquence présentant une identité de séquence d'au moins 50 %, 60 %, 70 %, 89 %, 90 % ou 95 % avec la SEQ ID n° : 70 et/ou 72, de préférence ledit anticorps humanisé comprenant la SEQ ID n° : 70 et/ou 72, et/ou CH1 et CH2 de SEQ ID n° : 70 ou un variant conservateur de l'une quelconque parmi les séquences précédentes ou une séquence présentant une identité de séquence d'au moins 50 %, 60 %, 70 %, 89 %, 90 % ou 95 % avec l'une quelconque des séquences précédentes.

**5.** Anticorps humanisé selon l'une quelconque des revendications 1 à 4, ledit anticorps humanisé étant un anticorps à chaîne unique.

**6.** Immunoconjugué comprenant l'anticorps humanisé selon l'une quelconque des revendications 1 à 5 et un marqueur détectable ou un agent cytotoxique.

**7.** Anticorps humanisé selon l'une quelconque des revendications 1 à 5 ou immunoconjugué selon la revendication 6, destiné à être utilisé dans le traitement de la maladie d'Alzheimer, la démence à corps de Lewy et/ou l'angiopathie amyloïde cérébrale.

**8.** Composition comprenant l'anticorps humanisé selon l'une quelconque des revendications 1 à 5 ou l'immunoconjugué selon la revendication 6 et un diluant.

**9.** Molécule d'acide nucléique codant l'anticorps humanisé selon l'une quelconque des revendications 1 à 5, de préférence ledit acide nucléique étant compris dans un vecteur.

**10.** Cellule exprimant l'anticorps humanisé selon l'une quelconque des revendications 1 à 5.

**11.** Immunoconjugué selon la revendication 6 destiné à être utilisé dans la mesure d'un taux de bêta-amyloïde in vivo, chez un sujet à risque ou soupçonné d'être atteint ou étant atteint de la maladie d'Alzheimer, ledit anticorps étant conjugué à un marqueur détectable, de préférence ledit marqueur étant un radionucléotide émetteur de positrons.

**12.** Quantité efficace d'une composition pharmaceutique comprenant l'anticorps humanisé ou l'immunoconjugué selon l'une quelconque des revendications 1 à 5 ou 6, destinée à être utilisée dans le traitement de la maladie d'Alzheimer, la démence à corps de Lewy et/ou l'angiopathie amyloïde cérébrale chez un sujet en ayant besoin.

**13.** Quantité efficace de l'anticorps humanisé ou de l'immunoconjugué selon l'une quelconque des revendications 1 à 5 ou 6, ou composition pharmaceutique destinée à être utilisée selon la revendication 12, ledit anticorps humanisé ou immunoconjugué étant administré directement au cerveau ou à une autre partie du SNC.

**14.** Utilisation de l'anticorps humanisé ou de l'immunoconjugué selon l'une quelconque des revendications 1 à 5 ou 6, ou d'une composition pharmaceutique comprenant ledit anticorps humanisé ou immunoconjugué, dans l'évaluation de la présence ou de taux de bêta-amyloïde dans un échantillon biologique prélevé chez un sujet devant être traité avec l'anticorps humanisé, l'immunoconjugué ou la composition pharmaceutique de ceux-ci selon les revendications 1 à 5 ou 6.

## Fig. 1

## Fig. 2

**Fig. 3A**
**Aducanumab**

**Fig. 3B**
**Bapineuzumab**

**Fig. 3C**
**IgG1 isotype control**

**Fig. 3D**
**Humanized 301-17**

**Fig. 3E**
**Mu 301-17**

**Fig. 3F**
**IgG4 isotype control**

## Fig. 4a

## Fig. 4b

## Fig. 4c

## Fig. 4d

**Fig. 5a**

**Equivalent amounts of aducanumab & hu 310-17 in brain and plasma at 24 hrs**

● Brain    ▲ Plasma

**Fig. 5b**

**Equivalent CNS penetrance of aducanumab & hu 310-17 at 24 hrs**

CNS penetrance

## Fig. 5c

### Non-Tg

## Fig. 5d

### APP/PS1

## Fig. 5e

### Brain/plasma ratio

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009048538 A2
- US 9221812 B2
- WO 2003070760 A2
- US 20110171243 A1
- WO 2008088983 A1
- WO 2001062801 A2
- WO 2009149487 A2
- US 20150105344 A1
- WO 2006125324 A1
- US 5766846 A
- US 5837672 A
- US 5593846 A
- WO 0162801 A
- WO 2004071408 A
- WO 2009086539 A2
- WO 2003070760 A
- WO 2006066089 A
- WO 2007068429 A
- WO 03016466 A
- WO 2017079833 A1
- EP 0239400 B1
- WO 9117271 A, Dower
- WO 9201047 A, McCafferty
- US 6150584 A
- US 6114598 A
- US 5770429 A
- US 2002076394 A, Leone
- US 5814014 A, Elsberry
- US 20060129126 A
- US 7012061 B

### Non-patent literature cited in the description

- **KARLIN ; ALTSCHUL.** *Proc. Natl. Acad. Sci. U.S.A.,* 1990, vol. 87, 2264-2268
- **KARLIN ; ALTSCHUL.** *Proc. Natl. Acad. Sci. U.S.A.,* 1993, vol. 90, 5873-5877
- **ALTSCHUL et al.** *J. Mol. Biol.,* 1990, vol. 215, 403
- **ALTSCHUL et al.** *Nucleic Acids Res.,* 1997, vol. 25, 3389-3402
- **MYERS ; MILLER.** *CABIOS,* 1988, vol. 4, 11-17
- Current Protocols in Molecular Biology. John Wiley & Sons, 2002
- **SAMBROOK et al.** Molecular Cloning: a Laboratory Manual. Cold Spring Harbor Laboratory Press, 2001
- **KOHLER ; MILSTEIN.** *Nature,* 1975, vol. 256, 495-497
- **KOZBOR et al.** *Immunol.Today,* 1983, vol. 4, 72
- **COLE et al.** *Methods Enzymol,* 1986, vol. 121, 140-67
- **HUSE et al.** *Science,* 1989, vol. 246, 1275
- **WARD et al.** *Nature,* 1989, vol. 41, 544-546
- **HUSE et al.** *Science,* 1989, vol. 246, 1275-1281
- **MCCAFFERTY et al.** *Nature,* 1990, vol. 348, 552-554
- **CARTER ; MERCHANT.** *Curr Opin Biotechnol,* 1997, vol. 8, 449-454
- **RIECHMANN et al.** *Nature,* 1988, vol. 332, 323-327
- **FOOTE ; WINTER.** *J Mol Biol,* 1992, vol. 224, 487-499
- **CO et al.** *J Immunol,* 1994, vol. 152, 2968-2976
- **PEDERSEN et al.** *J Mol Biol,* 1994, vol. 235, 959-973
- **JESPERS et al.** *Bio/Technology,* 1994, vol. 12, 899-903
- *Sci Transl Med.,* 20 July 2016, vol. 8 (348), 348ra96
- **GABRIELA A. N. CRESPI ; STEFAN J. HERMANS ; MICHAEL W. PARKER ; LUKE A. MILES.** Molecular basis for mid-region amyloid-b capture by leading Alzheimer's disease immunotherapies. *SCIENTIFIC REPORTS,* 2015, vol. 5, 9649
- **VINCENT J. HILSER ; ERNESTO FREIRE.** Structure-based calculation of the equilibrium folding pathway of proteins. correlation with hydrogen exchange protection factors. *J. Mol. Biol,* 1996, vol. 262, 756-772
- **SAMUEL I. A. COHEN.** Proliferation of amyloid-β42 aggregates occurs through a secondary nucleation mechanism. *Proc. Natl.I Acad. Sci. USA,* 2013, vol. 110 (24), 9758-9763

- **PIETRO SORMANNI ; FRANCESCO A. APRILE ; MICHELE VENDRUSCOLO.** The camsol method of rational design of protein mutants with enhanced solubility. *J of Mol Biol,* 2015, vol. 427 (2), 478-490
- **DEBORAH BLACKER ; MARILYN S. ALBERT ; SUSAN S. BASSETT ; RODNEY C. P. GO ; LINDY E. HARRELL ; MARSHAI F. FOLSTEIN.** Reliability and Validity of NINCDS-ADRDA Criteria for Alzheimer's Disease The National Institute of Mental Health Genetics Initiative. *Arch Neurol.,* 1994, vol. 51 (12), 1198-1204
- **HAMLEY, I.W.** *PEG-Peptide Conjugates,* 2014, vol. 15, 1543-1559
- **ROBERTS, MJ et al.** *Chemistry for peptide and protein PEGylation,* vol. 64, 116-127
- **J.X.LU ; W.QIANG ; W.M.YAU ; C.D.SCHWIETERS ; S.C.MEREDITH ; R.TYCKO.** MOLECULAR STRUCTURE OF BETA-AMYLOID FIBRILS IN AD BRAIN TISSUE. *CELL,* 2013, vol. 154, 1257
- **Y.XIAO ; B.MA ; D.MCELHENY ; S.PARTHASARATHY ; F.LONG ; M.HOSHI ; R.NUSSINOV ; Y.ISHII ; A BETA.** FIBRIL STRUCTURE ILLUMINATES SELF-RECOGNITION AND REPLICATION OF AMYLOID IN ALZHEIMER'S DISEASE. *NAT.STRUCT.MOL.BIOL.,* 2015, vol. 22 (1-42), 499
- **A.PETKOVA ; W.YAU ; R.TYCKO.** *EXPERIMENTAL CONSTRAINTS ON QUATERNARY STRUCTURE IN ALZHEIMER'S BETA-AMYLOID FIBRILS BIOCHEMISTRY,* 2006, vol. 45, 498
- **GIULIAN D ; HAVERKAMP LJ ; YU J ; KARSHIN W ; TOM D ; LI J ; KAZANSKAIA A ; KIRKPATRICK J ; ROHER AE.** The HHQK domain of β-amyloid provides a structural basis for the immunopathology of Alzheimer's disease. *J.BiolChem.,* 1998, vol. 273 (45), 29719-26
- **WINKLER K ; SCHARNAGL H ; TISLJAR U ; HOSCHÜTZKY H ; FRIEDRICH I ; HOFFMANN MM ; HÜTTINGER M ; WIELAND H ; MÄRZ W.** Competition of Aβ amyloid peptide and apolipoprotein E for receptor-mediated endocytosis. *J.Lipid Res.,* 1999, vol. 40 (3), 447-55
- *SCIENTIFIC REPORTS,* vol. 5, 9649
- **YU YZ ; WANG WB ; CHAO A ; CHANG Q ; LIU S ; ZHAO M et al.** Strikingly reduced amyloid burden and improved behavioral performance in Alzheimer's disease mice immunized with recombinant chimeric vaccines by hexavalent foldable Ab 1-15 fused to toxin-derived carrier proteins. *J Alzheimer's Dis,* 2014, vol. 41, 243-60
- **WANG, HC ; YU, YZ ; LIU, S ; ZHAO, M ; Q XU.** Peripherally administered sera antibodies recognizing amyloid- _oligomers mitigate Alzheimer's disease-like pathology and cognitive decline in aged 3× Tg-AD mice. *Vaccine,* 2016
- **ZOLA SM ; MANZANARES CM ; CLOPTON P ; LAH JJ ; LEVEY AL.** A behavioral task predicts conversion to mild cognitive impairment and Alzheimer's disease. *Am J Alzheimer's Dis & other dementia,* 2013, vol. 28 (2), 179-184
- **PETERS SJ et al.** Engineering an Improved IgG4 Molecule with Reduced Disulfide Bond Heterogeneity and Increased Fab Domain Thermal Stability. *The Journal of Biological Chemistry,* 2012, vol. 287, 24525-24533
- **SEVIGNY J et al.** The antibody aducanumab reduces AD plaque in Alzheimer's disease. *Nature,* 2016, vol. 537, 50-56